# EUROPÄISCHE PATENTANMELDUNG

(11) **EP 4 325 507 A1**
(43) Veröffentlichungstag der Anmeldung: **21.02.2024**
(21) Anmeldenummer: 22190373.5
(22) Anmeldetag: 15.08.2022
(51) Int. Cl.: G16H 10/60, G16H 30/20, G16H 40/20

(54) **VERFAHREN ZUR ZUORDNUNG EINES MEDIZINISCHEN GERÄTS**

(71) Anmelder: Siemens Healthineers AG, 91301 Forchheim (DE)
(72) Erfinder: Forman, Christoph, 91080 Uttenreuth (DE); Grimm, Robert, 90441 Nürnberg (DE); Nittka, Mathias, 91083 Baiersdorf (DE); Stranjak, Armin, 91080 Uttenreuth (DE)
(74) Vertreter: Siemens Healthineers Patent Attorneys

(57) **Zusammenfassung**

Die vorliegende Erfindung betrifft eine Technik zur Zuordnung eines medizinischen Geräts aus einer Gruppe medizinischer Geräte zu einer Untersuchung eines Patienten. Ein Verfahren ist in einer Scheduler-Agenten-Vorrichtung (200) implementiert und umfasst ein Empfangen, von einer Patienten-Agenten-Vorrichtung (402), einer Patientenparameter enthaltenden Nutzungsanfrage zur Untersuchung eines Patienten mittels eines medizinischen Geräts aus der Gruppe; ein Bestimmen einer Eignung mindestens eines medizinischen Geräts aus der Gruppe zur Untersuchung basierend auf dem oder den Patientenparametern und einem oder mehreren Geräteparametern; ein Zuordnen eines als geeignet bestimmten medizinischen Geräts zur Nutzungsanfrage basierend auf dem oder den Patientenparametern und Geräteparametern; ein Senden einer Mitteilung an die Patienten-Agenten-Vorrichtung (402) hinsichtlich der Zuordnung; und ein Senden einer Mitteilung an eine dem zugeordneten medizinischen Gerät beigeordnete Gerät-Agenten-Vorrichtung (300-1; 300-2; 300-3; 300-4).

## Beschreibung

Die vorliegende Erfindung betrifft ein computer-implementiertes Verfahren zur Zuordnung eines medizinischen Geräts zu einer Untersuchung eines Patienten, wobei das medizinische Gerät aus einer Gruppe, insbesondere gleichartiger, medizinischer Geräte zugeordnet wird. Weiterhin betrifft die Erfindung eine Scheduler-Agenten-Vorrichtung, eine Gerät-Agenten-Vorrichtung, ein System umfassend die Scheduler-Agenten-Vorrichtung, eine Mehrzahl an Gerät-Agenten-Vorrichtungen, eine oder mehrere Patienten-Agenten-Vorrichtungen, ein Computerprogramm und ein computerlesbares Speichermedium.

Herkömmlicherweise betreibt eine größere radiologische Abteilung eines Krankenhauses oder allgemein ein Gesundheitsdienstleister eine Flotte von bildgebenden medizinischen Geräten, beispielsweise Magnetresonanz-Tomographie (MRT)-Scannern. Die MRT-Scanner in der Flotte sind üblicherweise nicht identisch, sondern können eine Vielzahl unterschiedlicher Hard- und Softwareversionen aufweisen, wie z. B. unterschiedliche Feldstärken und Leistungsspezifikationen, unterschiedliche Bildgebungsmodalitäten wie neurologische, vaskuläre oder orthopädische Bildgebungsanwendungen mit zugehörigen Spezialausstattungen wie beispielsweise Spulen und Softwarepaketen. Die MRT-Scanner oder MRT-Systeme können beispielsweise auf eine Routine-Bildgebung, klinische Forschung oder spezielle Zwecke wie Notfälle, intraoperative Eingriffe oder Bestrahlungsplanung spezialisiert sein.

Die Frage, welcher Patient zu welchem Scanner geleitet werden soll, ist eine höchst nicht-triviale Aufgabe: Je nach Bildgebungsanforderung für eine bestimmte diagnostische Fragestellung durch den Auftraggeber muss ein Scanner ausgewählt werden, der die entsprechenden Bildgebungsmöglichkeiten bietet (z.B. Hardware, Lizenzen, installierte Software einschließlich Sequenzen, Rekonstruktionsalgorithmen, und/oder Protokolle).

Ein weiterer Faktor sind interne und externe Richtlinien, wie z. B. Qualitätsstandards und/oder Kompatibilitätsanforderungen. Weitere zu berücksichtigende Faktoren sind der Standort der Scanner (z. B. ob sich der Scanner im selben Gebäude befinden muss oder an einem weiter entfernten Ort sein kann) und die Verfügbarkeit in Bezug auf die Auslastung oder die Betriebszeiten.

Die Aufgabe, einen Patienten für einen bestimmten Scanner einzuplanen, erfordert daher herkömmlicherweise hochqualifiziertes Personal, die Verfügbarkeit sehr komplexer Informationen über die Infrastruktur des Scanners und viel Zeit für die Erfassung der erforderlichen Informationen. Herkömmlicherweise kann dies zu einer suboptimalen Patientenbehandlung, einer nicht optimalen Auslastung der Scanner, zeitaufwändigen Planungsprozessen und/oder zu Planungsfehlern und in Folge zu sicherheitskritischen Situationen führen.

Die herkömmliche Patientenplanung basiert ferner auf institutionellen Regeln, die üblicherweise eine Vereinfachung darstellen, so dass die Scanner-Auslastung nicht optimal ist.

Der vorliegenden Erfindung liegt die Aufgabe zugrunde, eine automatisierte Zuordnung von (insbesondere bildgebenden) medizinischen Geräten zu (z.B. Untersuchungen von) Patienten zu ermöglichen, die optimiert ist sowohl hinsichtlich der Auslastung einer Vielzahl von (insbesondere bildgebenden) medizinischen Geräten als auch hinsichtlich patienten-spezifischer Anforderungen, insbesondere hinsichtlich einer technischen Ausstattung und/oder (insbesondere Bild-) Qualität des medizinischen Geräts sowie hinsichtlich einer Dringlichkeit der Untersuchung des (und/oder Nutzung des medizinischen Geräts durch den) Patienten und/oder hinsichtlich einer Mobilität des Patienten.

Diese Aufgabe wird jeweils durch einen Gegenstand nach den unabhängigen und nebengeordneten Ansprüchen gelöst. Vorteilhafte Ausführungsformen sind Gegenstand der abhängigen Ansprüche, der Beschreibung und der Zeichnungen.

Gemäß einem Verfahrensaspekt wird ein computer-implementiertes Verfahren zur Zuordnung eines (beispielsweise bildgebenden) medizinischen Geräts aus einer Gruppe medizinischer Geräte zu einer Untersuchung eines Patienten bereitgestellt. Das Verfahren ist in einer Scheduler-Agenten-Vorrichtung implementiert. Das Verfahren umfasst einen Schritt des Empfangens einer Nutzungsanfrage von einer Patienten-Agenten-Vorrichtung zur Untersuchung eines Patienten mittels eines medizinischen Geräts aus der Gruppe der medizinischen Geräte. Die Nutzungsanfrage umfasst mindestens einen Patientenparameter des Patienten. Das Verfahren umfasst ferner einen Schritt des Bestimmens einer Eignung mindestens eines medizinischen Geräts aus der Gruppe der medizinischen Geräte zur Untersuchung basierend auf dem mindestens einen empfangenen Patientenparameter und mindestens einem Geräteparameter des medizinischen Geräts. Das Verfahren umfasst ferner einen Schritt des Zuordnens eines des mindestens einen als geeignet bestimmten medizinischen Geräts zu der empfangenen Nutzungsanfrage zur Untersuchung des Patienten basierend auf dem mindestens einen empfangenen Patientenparameter und dem mindestens einen Geräteparameter. Das Verfahren umfasst ferner einen Schritt des Sendens einer Mitteilung an die Patienten-Agenten-Vorrichtung. Die Mitteilung umfasst das zugeordnete als geeignet bestimmte medizinische Gerät zur Untersuchung des Patienten. Das Verfahren umfasst ferner einen Schritt des Sendens einer weiteren Mitteilung an eine dem zugeordneten medizinischen Gerät beigeordnete Gerät-Agenten-Vorrichtung. Die weitere Mitteilung umfasst die zugeordnete Untersuchung des Patienten.

Die Zuordnung zwischen medizinischem Gerät und Patient kann auch als Zuweisung (beispielsweise des Patienten zum medizinischen Gerät, oder umgekehrt) bezeichnet werden.

Das medizinische Gerät, oder mindestens eines oder jedes medizinische Gerät aus der Gruppe medizinischer Geräte, kann ein bildgebendes Gerät zur Ausführung eines bildgebenden Verfahrens umfassen, beispielsweise ein Gerät für Magnetresonanz-Tomographie (MRT), für Computer-Tomographie (CT), für Röntgenaufnahmen, für Ultraschallaufnahmen, und/oder für Positron-Emissions-Tomographie (PET), insbesondere für Einzelphoton-Emissions-Tomographie (englisch: single photon emission computer tomograph; kurz: SPECT). Jede Abkürzung eines bildgebenden Verfahrens kann, insbesondere je nach Kontext, einer Abkürzung für das entsprechende Gerät entsprechen. Beispielsweise kann MRT sowohl das Verfahren der Magnetresonanz-Tomographie als auch das Gerät zur Ausführung des Verfahrens, einen Magnetresonanz-Tomographen, bezeichnen.

Das medizinische Gerät kann zur Kombination von zwei oder mehr bildgebenden Verfahren ausgebildet sein, beispielsweise für eine Kombination von PET und CT, und/oder für eine Kombination von PET und MRT.

Die Gruppe medizinischer Geräte kann auch als Flotte medizinischer Geräte (kurz auch: Flotte) bezeichnet werden.

Die Gruppe medizinischer Geräte kann eine Vielzahl von medizinischen Geräten, insbesondere einer vorbestimmten Art (wobei die Art des medizinischen Geräts auch als Gerätetyp bezeichnet werden kann), an einem Standort und/oder in einem vorbestimmten Gebiet umfassen. Der Standort kann beispielsweise eine medizinische Einrichtung, insbesondere ein Krankenhaus, einen Krankenhaus-Campus und/oder ein Ärztehaus, umfassen. Alternativ oder ergänzend kann das vorbestimmte Gebiet beispielsweise eine Stadt, einen Landkreis und/oder ein Gebiet eines organisatorischen Zusammenschlusses mehrerer medizinischer Einrichtungen umfassen.

Die Vielzahl von medizinischen Geräten in der Gruppe medizinischer Geräte kann beispielsweise zwei bis fünfzig medizinische Geräte an einem Standort, und/oder bis zu mehreren hundert medizinischen Geräten in einem vorbestimmten Gebiet, beispielsweise in einer Großstadt, umfassen.

Die Untersuchung des Patienten kann ein Ausführen eines bildgebenden Verfahrens mittels des entsprechenden bildgebenden Geräts umfassen.

Der mindestens eine Patientenparameter des Patienten kann beispielsweise eine Größe, ein Gewicht, ein Alter, eine zu untersuchende Körperstelle und/oder das Vorhandensein eines Implantats umfassen. Alternativ oder ergänzend sind zu den Patientenparametern auch medizinische Diagnosen, wie z.B. der Verdacht auf Fraktur eines Wirbelkörpers und/oder entsprechende bevorzugte diagnostische Prozeduren, wie z.B. eine Bildgebung (z.B. das MRT) der Wirbelsäule, zu zählen.

Der mindestens eine Geräteparameter kann beispielsweise eine Magnetfeldstärke und/oder das Vorhandensein von Antennenspulen für zu untersuchende Körperstellen für ein MRT, und/oder eine Anzahl von Röntgenquellen eines CT, umfassen.

Dem Schritt des Zuordnens kann ein Schritt des Sendens einer Verfügbarkeitsanfrage und/oder des Empfangens einer Verfügbarkeitsauskunft vorangehen.

Die Schritte des Sendens der Mitteilung an die Patienten-Agenten-Vorrichtung und des Sendes der weiteren Mitteilung an die Gerät-Agenten-Vorrichtung können unabhängig voneinander und/oder in beliebiger Reihenfolge ausgeführt werden.

Der Schritt des Zuordnens kann ein Optimieren umfassen.

Mittels des computer-implementierten Verfahrens kann eine Auslastung und/oder eine gleichmäßige Nutzung der medizinischen Geräte in der Gruppe medizinischer Geräte optimiert werden. Alternativ oder ergänzend kann eine technische Eignung des medizinischen Geräts zur Untersuchung des Patienten optimiert werden. Aufgrund der optimierten technischen Eignung kann eine verbesserte Diagnose und/oder ein verbessertes Ergebnis der Untersuchung des Patienten ermöglicht werden. Weiterhin alternativ oder ergänzend kann mittels des Verfahrens ein Zeitfenster zur Untersuchung des Patienten optimiert werden, beispielsweise kann die Untersuchung zu einem frühestmöglichen Zeitpunkt durchgeführt werden, zu dem eines der Geräte aus der Gruppe medizinischer Geräte verfügbar und für die Untersuchung geeignet ist.

Das computer-implementierte Verfahren kann eine, insbesondere optimierte, Zuordnung der Untersuchung des Patienten zu einem medizinischen Gerät innerhalb einer kurzen Zeitspanne, beispielsweise in wenigen Minuten, ermöglichen.

Die Gruppe medizinischer Geräte kann eine Art medizinischer Geräte für eine vorbestimmte Art von Untersuchungen von Patienten umfassen.

Das medizinische Gerät kann ein bildgebendes Gerät umfassen. Alternativ oder ergänzend kann die Gruppe medizinischer Geräte eine Gruppe bildgebender Geräte umfassen.

Das bildgebende Gerät, und/oder die Gruppe bildgebender Geräte, kann einen Magnetresonanz-Tomographen (MRT) umfassen. Alternativ oder ergänzend kann das bildgebende Gerät, und/oder die Gruppe bildgebender Geräte, einen Computer-Tomographen (CT) umfassen. Alternativ oder ergänzend kann das bildgebende Gerät, und/oder die Gruppe bildgebender Geräte, ein Röntgengerät umfassen. Alternativ oder ergänzend kann das bildgebende Gerät, und/oder die Gruppe bildgebender Geräte, ein Ultraschallgerät umfassen. Alternativ oder ergänzend kann das bildgebende Gerät, und/oder die Gruppe bildgebender Geräte, einen Positronen-Emissions-Tomographen (PET) umfassen, insbesondere einen Einzelphotonen-Emissions-Tomographen (SPECT). Weiterhin alternativ oder ergänzend kann das bildgebende Gerät, und/oder die Gruppe bildgebender Geräte, eine Kombination von zwei oder mehr bildgebenden Geräten, insbesondere eine Kombination von PET und CT, und/oder eine Kombination von PET und MRT, umfassen.

Das Verfahren ferner einen Schritt des Sendens einer Verfügbarkeitsanfrage an mindestens eines des mindestens einen zur Eignung bestimmten medizinischen Geräts umfassen. Die Verfügbarkeitsanfrage kann an die dem jeweiligen zur Eignung bestimmten medizinischen Gerät beigeordnete Gerät-Agenten-Vorrichtung gesendet werden.

Alternativ oder ergänzend kann das Verfahren einen Schritt des Empfangens einer Verfügbarkeitsauskunft des mindestens einen zur Eignung bestimmten medizinischen Geräts umfassen, beispielsweise in Reaktion auf die Verfügbarkeitsanfrage. Die Verfügbarkeitsauskunft kann von der dem jeweiligen zur Eignung bestimmten medizinischen Gerät beigeordnete Gerät-Agenten-Vorrichtung empfangen werden.

Das Senden der Verfügbarkeitsanfrage kann die Frage umfassen, ob das medizinische Gerät betriebsbereit ist, und/oder ob das medizinische Gerät bereits anderweitig verplant und/oder für eine weitere Untersuchung eines weiteren Patienten verwendet wird.

Das Empfangen der Verfügbarkeitsauskunft kann in Antwort auf die Verfügbarkeitsanfrage erfolgen. Alternativ oder ergänzend kann die Verfügbarkeitsauskunft eine von der Gerät-Agenten-Vorrichtung initiierte Verfügbarkeitsauskunft umfassen.

Die von der Gerät-Agenten-Vorrichtung initiierte Verfügbarkeitsauskunft kann eine periodische Auskunft umfassen, beispielsweise ein periodisches "Ping" zur Mitteilung einer Betriebsbereitschaft des medizinischen Geräts. Alternativ oder ergänzend kann die von der Gerät-Agenten-Vorrichtung initiierte Verfügbarkeitsauskunft eine ereignisorientierte Auskunft umfassen. Die ergebnisorientierte Auskunft kann einen Leerlauf und/oder eine freie Verfügbarkeit des medizinischen Geräts umfassen. Alternativ oder ergänzend kann die ereignisorientierte Auskunft eine Fehlermeldung und/oder eine Meldung einer Nicht-Verfügbarkeit umfassen.

Alternativ oder ergänzend zu den Schritten des Sendens einer Verfügbarkeitsanfrage und/oder des Empfangens einer Verfügbarkeitsauskunft kann das Verfahren die Verwendung eines Watchdog-Timers umfassen. Ein Watchdog-Timer kann dazu ausgebildet sein, zu überprüfen, ob in festgelegten Zeitfenstern und/oder in festgelegten Zeitintervallen eine Verfügbarkeitsauskunft des medizinischen Geräts empfangen wird. Ein Nicht-Empfangen und/oder ein Ausbleiben einer Verfügbarkeitsauskunft, insbesondere nach Ablauf des festgelegten Zeitfensters und/oder des festgelegten Zeitintervalls kann einen Fehlerfall und/oder eine Nicht-Verfügbarkeit des medizinischen Geräts anzeigen und/oder indizieren.

Aufgrund einer festgestellten Änderung einer Verfügbarkeit eines medizinischen Geräts kann eine im Vorhinein bestimmte Zuordnung geändert werden. Alternativ oder ergänzend können die Schritte des Empfangens der Nutzungsanfrage und/oder der Bestimmung der Eignung des medizinischen Geräts (und/oder des mindestens einen Geräteparameters) einmalig ausgeführt werden, während nachfolgende Schritte betreffend die Zuordnung zwischen Patienten und medizinischen Geräten mehrfach ausgeführt werden können, beispielsweise aufgrund einer Änderung in der Verfügbarkeit mindestens eines medizinischen Geräts aus der Gruppe medizinischer Geräte.

Der mindestens eine Patientenparameter kann eine medizinische Fragestellung umfassen. Alternativ oder ergänzend kann der mindestens eine Patientenparameter eine zu untersuchende Körperstelle des Patienten umfassen. Alternativ oder ergänzend kann der mindestens eine Patientenparameter einen Körperbau des Patienten umfassen, insbesondere eine Größe und/oder ein Gewicht. Alternativ oder ergänzend kann der mindestens eine Patientenparameter ein Vorhandensein eines Implantats an oder nahe der zu untersuchenden Körperstelle des Patienten umfassen (beispielsweise einen Herzschrittmacher bei Untersuchungen des Rumpfs und/oder Brustkorb-Bereichs). Alternativ oder ergänzend kann der mindestens eine Patientenparameter ein Alter des Patienten umfassen. Alternativ oder ergänzend kann der mindestens eine Patientenparameter ein Geschlecht des Patienten oder der Patientin umfassen. Alternativ oder ergänzend kann der mindestens eine Patientenparameter eine Mobilität des Patienten umfassen. Alternativ oder ergänzend kann der mindestens eine Patientenparameter eine psychische Fitness des Patienten umfassen. Alternativ oder ergänzend kann der mindestens eine Patientenparameter eine physische Fitness des Patienten umfassen. Alternativ oder ergänzend kann der mindestens eine Patientenparameter eine Dringlichkeit der Untersuchung des Patienten umfassen. Alternativ oder ergänzend kann der mindestens eine Patientenparameter eine Anforderung an einen Geräteparameter umfassen, beispielsweise eine Anforderung an eine Magnetfeldstärke eines MRT. Weiterhin alternativ oder ergänzend kann der mindestens eine Patientenparameter eine Teilnahme an einer klinischen Studie umfassen.

Der mindestens eine Patientenparameter kann zwei oder mehr Patientenparameter umfassen, beispielsweise eine Kombination aus der zu untersuchenden Körperstelle, der Mobilität und der Dringlichkeit der Untersuchung des Patienten, und/oder der medizinischen Fragestellung, z.B. der Frage nach einem Bänderriss durch einen überweisenden Arzt oder eine andere medizinische Einrichtung. Alternativ oder ergänzend kann die zu untersuchende Körperstelle durch die medizinische Fragestellung konkretisiert werden. Beispielsweise kann die zu untersuchende Körperstelle ein Kniegelenk umfassen und die medizinische Fragestellung, ob ein Bänderriss am Kniegelenk vorliegt oder nicht. Die medizinische Fragestellung bzw. Diagnose können hierbei insbesondere auch als "Diagnostic Related Groups"-Code (kurz: DRG-Code, z.B. abrufbar über https://www.icd-code.de/icd/code/ICD-10-GM.html), "International Classification of Diseases"-Code (kurz: ICD-10-Code, z.B. abrufbar über https://www.icd-code.de/icd/code/ICD-10-GM.html) und/oder einen "Current Procedural Terminology"-Code (kurz: CPT-Code, z.B. abrufbar über https://www.cms.gov/license/ama?file=/files/zip/list-codes-effective-january-1-2022-published-november-19-2021.zip) repräsentiert sein. Beispielsweise wird mit dem ICD-10-Code M99.23 eine Sublexationsstenose des Spinalkanals, Lumbalbereich [lumbosakral], als biomechanische Funktionsstörung klassifiziert. Alternativ oder ergänzend steht der CPT-Code 72159 für "MR angio spine w/o&w/dye" (deutsch: "MR Angiographie Wirbelsäule ohne Kontrastmittel gefolgt von mit Kontrastmittel").

Die zu untersuchende Körperstelle kann den Kopf, den Hals, die Wirbelsäule, den Abdomen, und/oder ein oder mehrere Gelenke (z.B. ein Kniegelenk) des Patienten umfassen. Alternativ oder ergänzend kann die zu untersuchende Körperstelle klassifiziert werden, beispielsweise als neurologisch, auf ein oder mehrere Gefäße (beispielsweise mindestens eine Blutbahn und/oder ein oder mehrere Adern) bezogen, und/oder orthopädisch. Weiterhin alternativ oder ergänzend können eine oder mehrere bevorzugte diagnostische Prozeduren beispielsweise durch Angabe der entsprechenden CPT-Codes definiert werden.

Der Körperbau kann ein Gewicht des Patienten umfassen, beispielsweise ein Normalgewicht, Untergewicht und/oder Übergewicht. Alternativ oder ergänzend kann der Körperbau eine Größe und/oder eine Breite des Patienten umfassen.

Der Körperbau des Patienten kann die Zuordnung eines medizinischen Geräts umfassend eine Röhre und/oder einen Tunnel einschränken oder verhindern.

Das Implantat kann metallhaltig sein. Durch das Vorhandensein eines metallhaltigen Implantats kann die Zuordnung eines medizinischen Geräts eingeschränkt oder verhindert werden.

Das Implantat kann beispielsweise einen Herzschrittmacher, einen Stent, ein künstliches Gelenk (z.B. ein künstliches Kniegelenk und/oder ein künstliches Hüftgelenk), und/oder ein Zahnimplantat umfassen.

Das Alter des Patienten kann eine Zuordnung des medizinischen Geräts beschränken (z.B. durch medizinische Richtlinien und/oder gesetzliche Vorgaben). Beispielsweise kann eine maximale Röntgenstrahldosis für ein Kind beschränkt sein.

Die Mobilität des Patienten kann eine körperliche Fitness des Patienten umfassen und/oder einen Gesundheitszustand. Beispielsweise kann ein Patient, der aufgrund eines Unfalls hinsichtlich möglicher Verletzungen untersucht werden soll, nicht zwischen verschiedenen medizinischen Standorten transportabel sein.

Die psychische Fitness des Patienten kann eine Klaustrophobie (auch: Raumangst und/oder Angst vor engen Räumen) umfassen. Alternativ oder ergänzend kann die psychische Fitness eine Gelassenheit, Nervosität und/oder Selbstkontrolle des Patienten umfassen.

Die physische Fitness des Patienten kann beispielsweise die Fähigkeit umfassen, über einen vorbestimmten Zeitraum (beispielsweise bis zu 15 Sekunden oder bis zu ein paar Minuten) die Luft anzuhalten.

Eine Dringlichkeit der Untersuchung kann einen, beispielsweise maximalen, Zeitrahmen zur Planung der Untersuchung umfassen. Beispielsweise kann die Dringlichkeit der Untersuchung eines Patienten nach einem Unfall und/oder bei einem Notfall eine direkte, bzw. bis maximal einige Stunden (z.B. zwei Stunden), Untersuchung umfassen. Alternativ oder ergänzend kann keine Kontrolluntersuchung als nicht dringlich eingestuft werden, beispielsweise kann die Untersuchung mehrere Tage oder mehrere Wochen im Voraus eingeplant werden.

Weiterhin alternativ oder ergänzend kann eine Dringlichkeit der Untersuchung des Patienten eine intra-operative Untersuchung mittels des medizinischen Geräts umfassen. Die Dringlichkeit der Untersuchung kann der Terminplanung der Operation entsprechen.

Die Anforderung an einen Geräteparameter kann eine Anforderung an einen Bildkontrast, an eine Abbildungsart, beispielsweise umfassend ein Kontrastmittel, an eine (insbesondere Magnet-) Feldstärke, und/oder an eine Röntgendosis umfassen.

Die Anforderung an die Röntgendosis kann eine bereits über einen vorbestimmten früheren Zeitraum empfangene Röntgendosis des Patienten umfassen und/oder durch diese begrenzt sein. Alternativ oder ergänzend kann die Röntgendosis durch eine Empfehlung, beispielsweise in Abhängigkeit des Alters und/oder Geschlechts, bestimmt sein.

Die Teilnahme an einer klinischen Studie kann zu einer Beschränkung der Geräteparameter, und/oder zu einer Zuordnung der Untersuchung des Patienten zu einer vorbestimmten Untergruppe der Gruppe medizinischer Geräte führen.

Der mindestens eine Geräteparameter kann einen Standort des medizinischen Geräts umfassen. Alternativ oder ergänzend kann der mindestens eine Geräteparameter eine Bauweise des medizinischen Geräts, insbesondere umfassend eine Röhre, halboffen und/oder gerüst-basiert, umfassen. Alternativ oder ergänzend kann der mindestens eine Geräteparameter eine Belastbarkeit und/oder Verfahrbarkeit einer Patientenliege umfassen.

Alternativ oder ergänzend kann der mindestens eine Geräteparameter das Vorhandensein von Spezialzubehör (beispielsweise von Antennenspulen für MRT) umfassen. Alternativ oder ergänzend kann der mindestens eine Geräteparameter eine Feldstärke, insbesondere eine Magnetfeldstärke, umfassen. Alternativ oder ergänzend kann der mindestens eine Geräteparameter eine Anzahl an Röntgenquellen und zugehörigen Detektoren umfassen. Alternativ oder ergänzend kann der mindestens eine Geräteparameter eine Bildqualität umfassen. Alternativ oder ergänzend kann der mindestens eine Geräteparameter ein Vorhandensein von Spezial-Software zur Durchführung und/oder Auswertung der Untersuchung umfassen. Alternativ oder ergänzend kann der mindestens eine Geräteparameter eine durchschnittliche Zeitdauer einer Untersuchungsart umfassen, die der Nutzungsanfrage entspricht. Alternativ oder ergänzend kann der mindestens eine Geräteparameter eine zeitliche Verfügbarkeit umfassen. Alternativ oder ergänzend kann der mindestens eine Geräteparameter das Vorhandensein von geschultem Personal umfassen. Weiterhin alternativ oder ergänzend kann der mindestens eine Geräteparameter eine Eignung für klinische Studien umfassen.

Der mindestens eine Geräteparameter kann zwei oder mehr Geräteparameter umfassen. Beispielsweise kann der mindestens eine Geräteparameter einen Standort, eine Magnetfeldstärke und das Vorhandensein von Antennenspulen eines MRT umfassen.

Der Standort des medizinischen Geräts kann einen Standort einer medizinischen Einrichtung, eines Gebäudes, eines Stockwerks und/oder einer Raumart, beispielsweise eines Operationssaals, umfassen.

Die Bauweise des medizinischen Geräts kann eine Röhre und/oder einen Tunnel umfassen. Alternativ oder ergänzend kann der mindestens eine Geräteparameter einen Tunneldurchmesser und/oder einen Durchmesser der Röhre umfassen.

Alternativ oder ergänzend kann die Bauweise des medizinischen Geräts halboffen sein und/oder eine Gantry, insbesondere eine verschiebliche Gantry (sliding gantry) umfassen.

Die Belastbarkeit der Patientenliege kann ein vorbestimmtes Maximalgewicht des Patienten, beispielsweise inklusive am Patienten zu befestigendes Spezialzubehörs, umfassen.

Alternativ oder ergänzend kann die Verfahrbarkeit der Patientenliege umfassen, ob die Patientenliege teilweise oder ganz in eine Röhre oder einen Tunnel verfahrbar ist, bzw. ob die Möglichkeit besteht, einen Patienten auf der Liege von einem Vorbereitungsraum in den Untersuchungsraum zu fahren, ohne dass eine Umlagerung des Patienten auf die Patientenliege des Gerätes notwendig ist.

Das Spezialzubehör kann auch als Hardware-Ausstattung bezeichnet werden. Das Spezialzubehör kann eine Kontrastmittelinjektionseinrichtung, einen optischen Stimulator, eine Herzschlag- oder Herzstrom-Messeinrichtung (z.B. einen Elektrokardiogramm, EKG, -sensor), eine Einrichtung für Organpunktionen, eine Einrichtung für Tumorbiopsien, eine Beatmungseinrichtung, eine Sedierungseinrichtung, und/oder eine Signalempfangsstelle für eine Körperstelle des Patienten umfassen. Die Signalempfangsstelle kann eine Lokalspule (auch: Antennenspule) für MRT umfassen.

Die Kontrastmittelinjektionseinrichtung kann zur Darstellung von Blutbahnen, insbesondere Arterien, mit Hilfe eines Kontrastmittels dienen. Die Darstellung durch Kontrastmittel kann auch als "Angiographie" (oder auch "Arteriographie") bezeichnet werden. Alternativ oder ergänzend kann bei einer Angiographie ein Kontrastmittel direkt in eine Blutbahn, insbesondere eine Arterie, gespritzt und gleichzeitig eine Röntgenaufnahme gemacht werden, um die Blutbahn, insbesondere die Arterie, im Röntgenbild sichtbar zu machen.

Die Einrichtung für Organpunktionen kann beispielsweise bei einem Ultraschallgerät als medizinischem Gerät eingesetzt werden. Sollten beispielsweise in einer Ultraschalluntersuchung (auch: sonografisch), einer CT, und/oder einer MRT verdächtige Strukturen in einem Organ, beispielsweise der Leber, Bauchspeicheldrüse und/oder Lymphknoten, des Patienten entdeckt werden, können diese unter Ultraschallkontrolle gezielt punktiert werden. So kann insbesondere eine Gewebeprobe aus dem Organ zur feingeweblichen Untersuchung entnommen werden.

Die Feldstärke kann eine Magnetfeldstärke eines MRT umfassen. Die Magnetfeldstärke des MRT kann beispielsweise bei 0,5 Tesla (kurz: T); 0,55 T; 1,5 T; 3 T; 7 T und/oder 9,4 T liegen. Eine Feldstärke von 0,5 T kann als Niederfeldstärke (englisch: low field) bezeichnet werden. Alternativ oder ergänzend können Feldstärken von mindestens 7 T als Feldstärken für Forschungszwecke bezeichnet werden.

Eine Anzahl an Röntgenquellen und zugehörigen Detektoren, insbesondere für CT, kann eins oder zwei sein. Ein CT mit einer Röntgenquelle und zugehörigem Detektor kann auch als "Single-Source CT" bezeichnet werden. Ein CT mit zwei Röntgenquellen und zugehörigen Detektoren kann auch als "Dual-Source CT" bezeichnet werden. Alternativ oder ergänzend kann eine (z.B. durchschnittliche) Zeitdauer einer Untersuchung mit zwei Röntgenquellen kürzer sein als eine entsprechende Untersuchung mit eine Röntgenquelle. Eine kürzere Untersuchungszeit kann insbesondere für Trauma-Patienten und/oder Patienten, die aufgrund eines Unfalls untersucht werden, vorteilhaft sein. Als Trauma kann beispielsweise ein Verkehrsunfall und/oder ein Sturz bezeichnet werden.

Eine Bildqualität, insbesondere eine örtliche Auflösung, kann mit dem Wert der Feldstärke, insbesondere der Magnetfeldstärke eines MRT, zunehmen.

Die Bildqualität kann eine örtliche Auflösung und/oder eine zeitliche Auflösung umfassen. Eine konventionelle örtliche Auflösung kann im Bereich von 0,3 mm bis 1,5 mm liegen, beispielsweise bei MRT. Alternativ oder ergänzend kann eine örtliche Auflösung bei hoher Feldstärke und/oder für Forschungszwecke im sub-mm Bereich liegen und/oder kleiner als ein Millimeter (mm) sein.

Alternativ oder ergänzend kann die Bildqualität ein (insbesondere geringes) Bildrauschen umfassen.

Als Bildrauschen kann eine Verschlechterung eines digitalen bzw. elektronisch aufgenommenen Bildes durch Störungen bezeichnet werden, die keinen Bezug zum eigentlichen Bildinhalt und/oder dem Bildsignal haben. Störende Pixel können beispielsweise in Farbe und/oder Helligkeit von denen des eigentlichen Bildes abweichen.

Die Spezial-Software kann ein Untersuchungsprotokoll und/oder ein Messprotokoll umfassen. Ein Untersuchungsprotokoll kann insbesondere eine Untersuchung beim Vorhandensein eines Implantats, beispielsweise als Patientenparameter, ermöglichen.

Alternativ oder ergänzend kann die Spezial-Software eine oder mehrere Sequenzen, insbesondere Messsequenzen, umfassen. Eine Messsequenz kann beispielsweise eine Anzahl und/oder Breite von (beispielsweise elektromagnetischen) Impulsen, eine Magnetfeldzuschaltung, und/oder ein Turbo-Spin-Echo (TSE) umfassen. Alternativ oder ergänzend kann die Messsequenz ein Spin-Echo (SE), ein Spin-Echo fat saturation (SE fs; deutsch: SE mit Fettsättigung), ein Fluid Attenuated Inversion Recovery (FLAIR; deutsch: Wassersättigung) und/oder Gradient-Echo (GRE) umfassen. Weiterhin alternativ oder ergänzend kann die Messsequenz, insbesondere für eine Schlaganfall-Diagnostik, ein Diffusion weighted imaging (DWI) und/oder Perfusion weighted imaging (PWI) umfassen. Weiterhin alternativ oder kann die Messsequenz ein Turbo-Inversion Recovery-Magnitude (TIRM), Short-Tau Inversion Recovery (STIR; deutsch: Fett-Signal Unterdrückung) und/oder Diffusion tensor imaging (DTI) umfassen.

Weiterhin alternativ oder ergänzend kann eine Spezial-Software geeignet sein zur Durchführung einer funktionellen MRT (kurz: fMRT; oder fMRI für englisch: functional magnetic resonance imaging), beispielsweise zur funktionellen Untersuchung des Gehirns. Mittels fMRT können physiologische Funktionen im Inneren des Körpers mit den Methoden der MRT dargestellt werden.

Die durchschnittliche Zeitdauer einer Untersuchungsart kann einem Erfahrungswert entsprechen und/oder zur Zeitplanung durch die Scheduler-Agenten-Vorrichtung verwendet werden.

Die zeitliche Verfügbarkeit des medizinischen Geräts kann eine Verfügbarkeit laut (z.B. bestehendem) Terminplan umfassen. Der Terminplan kann auch als Zeitplan bezeichnet werden.

Alternativ oder ergänzend kann die zeitliche Verfügbarkeit eine zeitlich begrenzte Nicht-Verfügbarkeit umfassen, beispielsweise aufgrund einer zeitlichen Verschiebung einer länger andauernden Untersuchung, einer Untersuchung eines Patienten (z.B. eines Notfallpatienten) entgegen eines (z.B. bestehenden) Terminplans. Alternativ oder ergänzend kann eine begrenzte Nicht-Verfügbarkeit umfassen, dass das medizinische Gerät außer Betrieb ist, beispielsweise aufgrund eines Defekts und/oder aufgrund von Wartungsarbeiten. Die Wartungsarbeiten können auch eine Erweiterung und/oder ein Upgrade (beispielsweise der Software, insbesondere der Spezial-Software) des medizinischen Geräts umfassen.

Das Vorhandensein von geschultem Personal kann aufwändigere Untersuchungen ermöglichen. Alternativ oder ergänzend kann das Vorhandensein von geschultem Personal, von Spezial-Software und/oder von Spezialzubehör eine Eignung für klinische Studien ermöglichen. Weiterhin alternativ oder ergänzend kann die Spezial-Software das geschulte Personal in der Planung der Untersuchung, in der Durchführung der Untersuchung und/oder in einem Arbeitsablauf unterstützen.

Die Eignung für klinische Studien als Geräteparameter kann sich auf eine oder mehrere Untersuchungsarten beziehen.

Der Schritt des Empfangens der Nutzungsanfrage kann eine Eingabe auf einer Benutzer-Schnittstelle und/oder eine Abfrage eines Datenbank-Systems umfassen. Die Abfrage des Datenbank-Systems kann eine Abfrage von einem oder mehreren Patientenparametern und/oder einer Vorgeschichte des Patienten umfassen.

Die Eingabe auf der Benutzer-Schnittstelle kann eine Eingabe auf einem Computer, Tablet, Smartphone und/oder einem anderen Benutzergerät umfassen.

Das Datenbanksystem kann ein Krankenhausinformationssystem (KIS) umfassen. Als KIS kann die Klasse der Gesamtheit aller informationsverarbeitenden Systeme der Informationstechnik zur Erfassung, Bearbeitung und/oder Weitergabe medizinischer und/oder administrativer Daten in einem Krankenhaus bezeichnet werden.

Alternativ oder ergänzend kann das Datenbanksystem ein Radiologie Informationssystem (RIS) umfassen. Als RIS kann die Klasse der Gesamtheit aller informationsverarbeitenden System der Informationstechnik zur Erfassung, Bearbeitung und/oder Weitergabe medizinischer und/oder administrativer Daten in einer Radiologie bezeichnet werden.

Eine Radiologie kann eine Vielzahl unterschiedlicher Arten bildgebender medizinischer Geräte umfassen, beispielsweise MRT, CT, Röntgengeräte, Ultraschall-Geräte und/oder PET.

Der Schritt des Zuordnens eines des mindestens einen als geeignet bestimmten medizinischen Geräts zu der empfangenen Nutzungsanfrage kann ein Optimieren einer Menge von Zuordnungen unterschiedlicher Patienten, umfassend den Patienten, zu medizinischen Geräten aus der Gruppe medizinischer Geräte umfassen.

Der Schritt des Optimierens kann eine Wichtung (auch: Gewichtung) von Patientenparametern und/oder Geräteparametern umfassen. Alternativ oder ergänzend kann das Zuordnen durch eine Übersetzungsmatrix, beispielsweise von Patientenparametern zu Geräteparametern, beschreibbar sein.

Die Zuordnung und/oder Optimierung der Menge von Zuordnungen kann konfigurierbar sein. Beispielsweise kann eine Konfiguration geändert werden, wenn ein medizinisches Gerät aus der Gruppe medizinischer Geräte, eingeschränkt (bzw. abgeschafft) und/oder erweitert wird und/oder ein neues medizinisches Gerät hinzukommt. Alternativ oder ergänzend kann eine Änderung der Konfiguration eine Änderung der zeitlichen Verfügbarkeit und/oder der durchschnittlichen Zeitdauer der Untersuchungsart umfassen.

Das Optimieren kann ein Minimieren einer Linearkombination dimensionsloser Kostenfunktionen umfassen. Jedem des mindestens einen Patientenparameter kann eine dimensionslose Kostenfunktion zugeordnet sein.

Die Kostenfunktion kann eine (insbesondere normierte) Abstandsfunktion von einer idealen Zuordnung des jeweiligen Patientenparameters zu einem oder mehreren Geräteparametern und/oder zu einem medizinischen Gerät umfassen.

Die Linearkombination der dimensionslosen Kostenfunktionen kann eine gewichtete Summe der dimensionslosen Kostenfunktionen umfassen.

Mittels der Gewichtung kann beispielsweise eine Dringlichkeit und/oder ein Standort für einen Trauma-Patienten wichtiger eingestuft werden als technische Spezifikationen, beispielsweise umfassend eine Magnetfeldstärke eines MRT.

Alternativ oder ergänzend können für bestimmte anatomische Regionen, beispielsweise kleinere Gelenke, höhere Magnetfeldstärken als wichtiger eingestuft werden und/oder ein höheres Gewicht erhalten (auch: höher gewichtet werden). Vorteilhafterweise kann dadurch eine höhere Auflösung, beispielsweise durch die Wahl eines entsprechenden medizinischen Geräts, erreicht werden.

Alternativ oder ergänzend können für vorbestimmte Patientengruppen, beispielsweise Kinder und/oder Schwangere, geringere Strahlenbelastungen wünschenswert und/oder gesetzlich vorgeschrieben sein. Beispielsweise kann ein Röntgengerät, das baubedingt eine geringere Strahlendosis aufweist, für die vorbestimmte Patientengruppe höher gewichtet werden.

Für anderweitige, beispielsweise stark standardisierte und/oder weniger anspruchsvolle, (insbesondere Routine-) Untersuchungen können Gewichte anders gewählt werden. Beispielsweise kann eine Energieeinsparung, eine gleichmäßige Auslastung der medizinischen Geräte, und/oder ein Freihalten von hochwertigen (insbesondere Forschungs-) Geräten mittels einer entsprechenden Gewichtung favorisiert und/oder erreicht werden.

Das Optimieren kann einen oder mehrere Schritte eines paarweisen Vertauschens von Zuordnungen eines Patienten zu einem medizinischen Gerät umfassen.

Das paarweise Vertauschen kann einer Iteration des Optimierens entsprechen. Das paarweise Vertauschen kann beispielsweise so lange fortgeführt werden, bis keine bessere Zuordnung mehr gefunden wird. Alternativ oder ergänzend kann das paarweise Vertauschen nach einer vorbestimmten Zeitdauer beendet werden.

Vorstehend wurde die Lösung der Aufgabe anhand des Verfahrens beschriebenen. Dabei erwähnte Merkmale, Vorteile oder alternative Ausführungsformen sind ebenso auch auf die anderen beanspruchten Gegenstände zu übertragen und umgekehrt. Mit anderen Worten können auch die gegenständlichen Ansprüche (die beispielsweise auf Agenten-Vorrichtungen, ein System oder auf ein Computerprogrammprodukt gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, der Agenten-Vorrichtung, des Systems bzw. des Produktes ausgebildet und umgekehrt.

Gemäß einem ersten Vorrichtungsaspekt wird eine Scheduler-Agenten-Vorrichtung zur Zuordnung eines medizinischen Geräts aus einer Gruppe medizinischer Geräte zu einer Untersuchung eines Patienten bereitgestellt. Die Scheduler-Agenten-Vorrichtung umfasst eine Empfangseinheit, die ausgebildet ist zum Empfangen einer Nutzungsanfrage zur Untersuchung eines Patienten mittels eines medizinischen Geräts aus der Gruppe der medizinischen Geräte. Die Nutzungsanfrage wird von einer Patienten-Agenten-Vorrichtung empfangen. Die Nutzungsanfrage umfasst mindestens einen Patientenparameter des Patienten.

Die Scheduler-Agenten-Vorrichtung umfasst ferner eine Bestimmungseinheit, die ausgebildet ist zum Bestimmen einer Eignung mindestens eines medizinischen Geräts aus der Gruppe der medizinischen Geräte zur Untersuchung basierend auf dem mindestens einen empfangenen Patientenparameter und mindestens einem Geräteparameter des medizinischen Geräts. Die Scheduler-Agenten-Vorrichtung umfasst ferner eine Zuordnungseinheit, die ausgebildet ist zum Zuordnen eines des mindestens einen als geeignet bestimmten medizinischen Geräts zu der empfangenen Nutzungsanfrage zur Untersuchung des Patienten basierend auf dem mindestens einen empfangenen Patientenparameter und dem mindestens einen Geräteparameter. Die Scheduler-Agenten-Vorrichtung umfasst ferner eine erste Sendeeinheit, die ausgebildet ist zum Senden einer Mitteilung umfassend das zugeordnete als geeignet bestimmte medizinische Gerät zur Untersuchung des Patienten an die Patienten-Agenten-Vorrichtung. Die Scheduler-Agenten-Vorrichtung umfasst ferner eine zweite Sendeeinheit, die ausgebildet ist zum Senden einer weiteren Mitteilung an eine dem zugeordneten medizinischen Gerät beigeordnete Gerät-Agenten-Vorrichtung. Die weitere Mitteilung umfasst die zugeordnete Untersuchung des Patienten.

Die Scheduler-Agenten-Vorrichtung kann ein jedes Merkmal umfassen, das im Zusammenhang mit dem Verfahren beschrieben ist.

Die Scheduler-Agenten-Vorrichtung kann alternativ oder ergänzend dazu ausgebildet sein, die Schritte des Verfahrensaspekts auszuführen.

Gemäß einem zweiten Vorrichtungsaspekt wird eine Gerät-Agenten-Vorrichtung zur Zuordnung eines medizinischen Geräts aus einer Gruppe medizinischer Geräte zu einer Untersuchung eines Patienten bereitgestellt. Die Gerät-Agenten-Vorrichtung ist dem medizinischen Gerät zugeordnet ist. Die Gerät-Agenten-Vorrichtung umfasst eine erste Empfangseinheit, die ausgebildet ist zum Empfangen einer Mitteilung umfassend die zugeordnete Untersuchung des Patienten. Die Mitteilung wird von einer Scheduler-Agenten-Vorrichtung empfangen.

Die Gerät-Agenten-Vorrichtung kann ferner eine zweite Empfangseinheit umfassen, die ausgebildet ist zum Empfangen einer Verfügbarkeitsanfrage betreffend das medizinische Gerät von der Scheduler-Agenten-Vorrichtung. Alternativ oder ergänzend kann die Gerät-Agenten-Vorrichtung eine Sendeeinheit umfassen, die ausgebildet ist zum Senden einer Verfügbarkeitsauskunft betreffend das medizinische Gerät an die Scheduler-Agenten-Vorrichtung.

Gemäß einem Systemaspekt wird ein System zur Zuordnung eines medizinischen Geräts aus einer Gruppe medizinischer Geräte zu einer Untersuchung eines Patienten bereitgestellt. Das System umfasst eine Scheduler-Agenten-Vorrichtung gemäß dem ersten Vorrichtungsaspekt, eine Vielzahl von Gerät-Agenten-Vorrichtungen gemäß dem zweiten Vorrichtungsaspekt, wobei jede Gerät-Agenten-Vorrichtung einem medizinischen Gerät zugeordnet ist, und mindestens eine Patienten-Agenten-Vorrichtung zur Zuordnung eines medizinischen Geräts aus einer Gruppe medizinischer Geräte zu einer Untersuchung eines Patienten. Die Patienten-Agenten-Vorrichtung umfasst eine Empfangseinheit, die ausgebildet ist zum Empfangen mindestens eines Patientenparameters zur Untersuchung eines Patienten. Die Patienten-Agenten-Vorrichtung umfasst ferner eine Sendeeinheit, die ausgebildet ist zum Senden einer Nutzungsanfrage zur Untersuchung des Patienten mittels eines medizinischen Geräts aus der Gruppe der medizinischen Geräte. Die Nutzungsanfrage wird an die Scheduler-Agenten-Vorrichtung gesendet. Die Nutzungsanfrage umfasst den mindestens einen Patientenparameter des Patienten.

Gemäß einem weiteren Aspekt wird ein Computerprogrammprodukt bereitgestellt. Das Computerprogrammprodukt umfasst Befehle, die bei der Ausführung des Programms durch einen Computer diesen veranlassen, das Verfahren gemäß dem Verfahrensaspekt auszuführen.

Gemäß einem weiteren Aspekt wird ein computerlesbares Speichermedium bereitgestellt. Das computerlesbare Speichermedium umfasst Befehle, die bei der Ausführung durch mindestens einen Computer diesen veranlassen, die Schritte des Verfahrens gemäß dem Verfahrensaspekt auszuführen.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: ein Ablaufdiagramm eines Verfahrens gemäß einer bevorzugten Ausführungsform der Erfindung;
- Fig. 2: ein Blockschaltbild einer Scheduler-Agenten-Vorrichtung gemäß einer bevorzugten Ausführungsform der Erfindung, wobei die Scheduler-Agenten-Vorrichtung zur Ausführung des Verfahrens gemäß Fig. 1 ausgebildet sein kann;
- Fig. 3: ein Blockschaltbild einer Gerät-Agenten-Vorrichtung gemäß einer bevorzugten Ausführungsform der Erfindung;
- Fig. 4: ein schematisches Beispiel eines Systems umfassend eine Scheduler-Agenten-Vorrichtung, beispielsweise gemäß Fig. 2, eine Mehrzahl Gerät-Agenten-Vorrichtungen, beispielsweise gemäß Fig. 3, und eine Mehrzahl Patienten-Agenten-Vorrichtungen;
- Fig. 5: ein erstes Ausführungsbeispiel des erfindungsgemäßen Verfahrens mit Zuordnung der Schritte zu den jeweiligen Agenten-Vorrichtungen;
- Fig. 6: ein zweites Ausführungsbeispiel des erfindungsgemäßen Verfahrens mit Zuordnung der Schritte zu den jeweiligen Agenten-Vorrichtungen;
- Fig. 7: ein Beispiel einer Zuordnung medizinischer Geräte zu Patienten in Abhängigkeit von einer Zeit der geplanten Untersuchung;
- Fig. 8: einen beispielhaften Schritt der Optimierung der Zuordnung zwischen einem medizinischen Gerät und einem Patienten in Abhängigkeit von der Zeit der geplanten Untersuchung; und
- Fig. 9: ein Ausführungsbeispiel der Optimierungsschritte der Zuordnung zwischen medizinischen Geräten und Untersuchungen von Patienten.

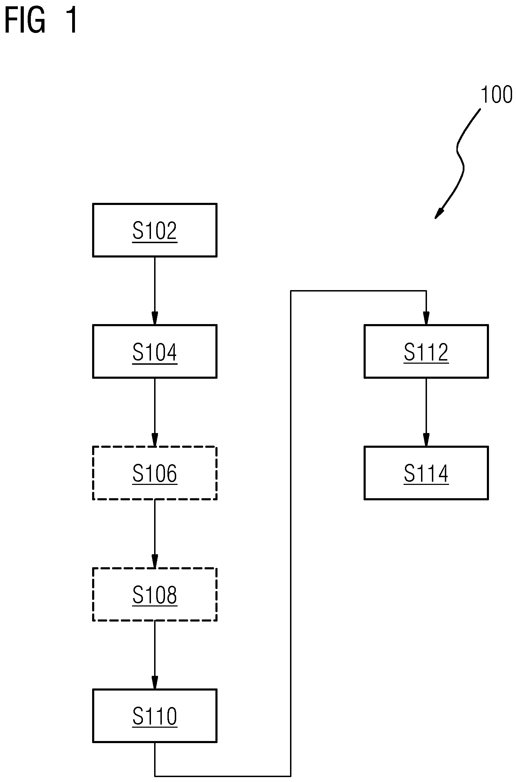

Fig. 1 zeigt schematisch ein Ausführungsbeispiel eines computer-implementiertes Verfahrens zur Zuordnung eines medizinischen Geräts aus einer Gruppe medizinischer Geräte zu einer Untersuchung eines Patienten. Das Verfahren ist allgemein mit Bezugszeichen 100 bezeichnet. Das Verfahren kann in einer Scheduler-Agenten-Vorrichtung implementiert sein.

Das Verfahren 100 umfasst einen Schritt S102 des Empfangens einer Nutzungsanfrage zur Untersuchung eines Patienten mittels eines medizinischen Geräts aus der Gruppe der medizinischen Geräte. Die Nutzungsanfrage wird von einer Patienten-Agenten-Vorrichtung empfangen und umfasst mindestens einen Patientenparameter des Patienten.

Das Verfahren 100 umfasst ferner einen Schritt S104 des Bestimmens einer Eignung mindestens eines medizinischen Geräts aus der Gruppe der medizinischen Geräte zur Untersuchung basierend auf dem mindestens einen empfangenen S102 Patientenparameter und mindestens einem Geräteparameter des medizinischen Geräts.

Das Verfahren 100 umfasst ferner einen Schritt S110 des Zuordnens eines des mindestens einen als geeignet bestimmten S104 medizinischen Geräts zu der empfangenen S102 Nutzungsanfrage zur Untersuchung des Patienten basierend auf dem mindestens einen empfangenen S102 Patientenparameter und dem mindestens einen Geräteparameter.

Das Verfahren 100 umfasst ferner einen Schritt S112 des Sendens einer Mitteilung umfassend das zugeordnete S110 als geeignet bestimmte S104 medizinische Gerät zur Untersuchung des Patienten. Die Mitteilung wird im Schritt S112 an die Patienten-Agenten-Vorrichtung gesendet.

Das Verfahren 100 umfasst ferner einen Schritt S114 des Sendens einer Mitteilung umfassend die zugeordnete S110 Untersuchung des Patienten. Die Mitteilung wird im Schritt S114 an eine dem zugeordneten S110 medizinischen Gerät beigeordnete Gerät-Agenten-Vorrichtung gesendet.

Optional umfasst das Verfahren 100 ferner einen Schritt S106 des Sendens einer Verfügbarkeitsanfrage an mindestens eines des mindestens einen zur Eignung bestimmten S104 medizinischen Geräts. Die Verfügbarkeitsanfrage kann an die dem jeweiligen zur Eignung bestimmten medizinischen Gerät beigeordnete Gerät-Agenten-Vorrichtung gesendet werden.

Weiterhin optional umfasst das Verfahren 100 einen Schritt S108; S108' des Empfangens einer Verfügbarkeitsauskunft des mindestens einen zur Eignung bestimmten S104 medizinischen Geräts. Die Verfügbarkeitsauskunft kann in Antwort auf die im Schritt S106 gesendete Verfügbarkeitsanfrage empfangen werden. Die Verfügbarkeitsauskunft kann von der dem jeweiligen zur Eignung bestimmten medizinischen Gerät beigeordnete Gerät-Agenten-Vorrichtung empfangen werden.

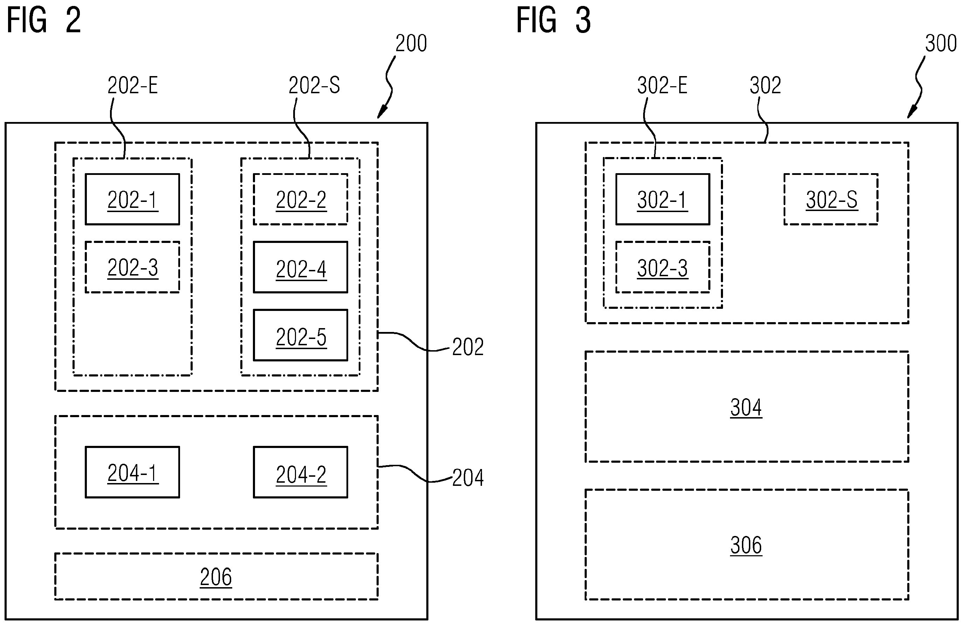

Fig. 2 zeigt schematisch ein Ausführungsbeispiel einer Scheduler-Agenten-Vorrichtung zur Zuordnung eines medizinischen Geräts aus einer Gruppe medizinischer Geräte zu einer Untersuchung eines Patienten. Die Scheduler-Agenten-Vorrichtung wird allgemein mit Bezugszeichen 200 bezeichnet.

Die Scheduler-Agenten-Vorrichtung 200 umfasst eine Empfangseinheit 202-1, die ausgebildet ist zum Empfange einer Nutzungsanfrage zur Untersuchung eines Patienten mittels eines medizinischen Geräts aus der Gruppe der medizinischen Geräte. Die Nutzungsanfrage kann von einer Patienten-Agenten-Vorrichtung empfangen werden und mindestens einen Patientenparameter des Patienten umfassen.

Die Scheduler-Agenten-Vorrichtung 200 umfasst ferner eine Bestimmungseinheit 204-1, die ausgebildet ist zum Bestimmen einer Eignung mindestens eines medizinischen Geräts aus der Gruppe der medizinischen Geräte zur Untersuchung basierend auf dem mindestens einen empfangenen Patientenparameter und mindestens einem Geräteparameter des medizinischen Geräts.

Die Scheduler-Agenten-Vorrichtung 200 umfasst ferner eine Zuordnungseinheit 204-2, die ausgebildet ist zum Zuordnen eines des mindestens einen als geeignet bestimmten medizinischen Geräts zu der empfangenen Nutzungsanfrage zur Untersuchung des Patienten basierend auf dem mindestens einen empfangenen Patientenparameter und dem mindestens einen Geräteparameter.

Die Scheduler-Agenten-Vorrichtung 200 umfasst ferner eine erste Sendeeinheit 202-4, die ausgebildet ist zum Senden einer Mitteilung umfassend das zugeordnete als geeignet bestimmte medizinische Gerät zur Untersuchung des Patienten. Die erste Sendeeinheit 202-4 ist dazu ausgebildet, die Mitteilung an die Patienten-Agenten-Vorrichtung zu senden.

Die Scheduler-Agenten-Vorrichtung 200 umfasst ferner eine zweite Sendeeinheit 202-5, die ausgebildet ist zum Senden einer Mitteilung umfassend die zugeordnete Untersuchung des Patienten. Die Sendeeinheit 202-5 ist dazu ausgebildet, die Mitteilung an eine dem zugeordneten medizinischen Gerät beigeordnete Gerät-Agenten-Vorrichtung zu senden.

Optional umfasst die Scheduler-Agenten-Vorrichtung 200 ferner eine weitere Sendeeinheit 202-2, die dazu ausgebildet ist, eine Verfügbarkeitsanfrage an mindestens eines der mindestens einen zur Eignung bestimmten medizinischen Geräte zu senden. Die Verfügbarkeitsanfrage kann an die dem jeweiligen zur Eignung bestimmten medizinischen Gerät beigeordnete Gerät-Agenten-Vorrichtung gesendet werden.

Weiterhin optional umfasst die Scheduler-Agenten-Vorrichtung 200 eine weitere Empfangseinheit 202-3, die dazu ausgebildet ist, eine Verfügbarkeitsauskunft des mindestens einen zur Eignung bestimmten medizinischen Geräts zu empfangen. Die Verfügbarkeitsauskunft kann in Antwort auf die gesendete Verfügbarkeitsanfrage empfangen werden. Die Verfügbarkeitsauskunft kann von der dem jeweiligen zur Eignung bestimmten medizinischen Gerät beigeordnete Gerät-Agenten-Vorrichtung empfangen werden.

Die Sendeeinheiten 202-4 und 202-5, und optional die weitere Sendeeinheit 202-2, können in einer gemeinsamen Sendeeinheit 202-S zusammengefasst sein. Alternativ oder ergänzend können die Empfangseinheit 202-1 und die optionale weitere Empfangseinheit 202-3 in einer gemeinsamen Empfangseinheit 202-E zusammengefasst sein. Weiterhin alternativ oder ergänzend können alle Sendeeinheiten 202-4, 202-5 (und optional 202-2) und Empfangseinheiten 202-1 (und optional 202-3) in einer gemeinsamen Sende-Empfangseinheit 202 vereint sein. Die gemeinsame Sende-Empfangseinheit 202 kann auch als Datenschnittstelle bezeichnet werden.

Optional sind ferner die Bestimmungseinheit 204-1 und die Zuordnungseinheit 204-2 der Scheduler-Agenten-Vorrichtung 200 in einer gemeinsamen Einheit 204, beispielsweise einem Prozessor, zusammengefasst.

Optional umfasst die Scheduler-Agenten-Vorrichtung 200 ferner eine Speichereinheit 206.

Fig. 3 zeigt schematisch ein Ausführungsbeispiel einer Gerät-Agenten-Vorrichtung zur Zuordnung eines medizinischen Geräts aus einer Gruppe medizinischer Geräte zu einer Untersuchung eines Patienten. Die Gerät-Agenten-Vorrichtung ist allgemein mit Bezugszeichen 300 bezeichnet. Die Gerät-Agenten-Vorrichtung 300 ist dem medizinischen Gerät zugeordnet.

Die Gerät-Agenten-Vorrichtung 300 umfasst eine erste Empfangseinheit 302-1, die ausgebildet ist zum Empfangen einer Mitteilung umfassend die zugeordnete Untersuchung des Patienten. Die Gerät-Agenten-Vorrichtung 300 ist dazu ausgebildet, die Mitteilung von einer Scheduler-Agenten-Vorrichtung, beispielsweise der Vorrichtung 200, zu empfangen.

Optional umfasst die Gerät-Agenten-Vorrichtung 300 eine zweite Empfangseinheit 302-3, die ausgebildet ist zum Empfangen einer Verfügbarkeitsanfrage betreffend das medizinische Gerät von der Scheduler-Agenten-Vorrichtung, beispielsweise der Vorrichtung 200. Die zweite Empfangseinheit 302-3 kann dazu ausgebildet sein, die Verfügbarkeitsanfrage von der Scheduler-Agenten-Vorrichtung, beispielsweise der Vorrichtung 200, zu empfangen.

Weiterhin optional umfasst die Gerät-Agenten-Vorrichtung 300 eine Sendeeinheit 302-S, die ausgebildet ist zum Senden einer Verfügbarkeitsauskunft betreffend das medizinische Gerät an die Scheduler-Agenten-Vorrichtung. Die Sendeeinheit 302-S kann dazu ausgebildet sein, die Verfügbarkeitsauskunft an die Scheduler-Agenten-Vorrichtung, beispielsweise die Vorrichtung 200, zu senden.

Die Empfangseinheiten 302-1 und 302-3 können in einer gemeinsamen Empfangseinheit 302-E zusammengefasst sein. Alternativ oder ergänzend können die Empfangseinheiten 302-1, 302-3 und die Sendeeinheit 302-S in einer gemeinsamen Sende-Empfangseinheit 302 zusammengefasst sein. Die gemeinsame Sende-Empfangseinheit 302 kann auch als Datenschnittstelle bezeichnet werden.

Optional umfasst die Gerät-Agenten-Vorrichtung 300 ferner einen Prozessor 304 und/oder eine Speichereinheit 306.

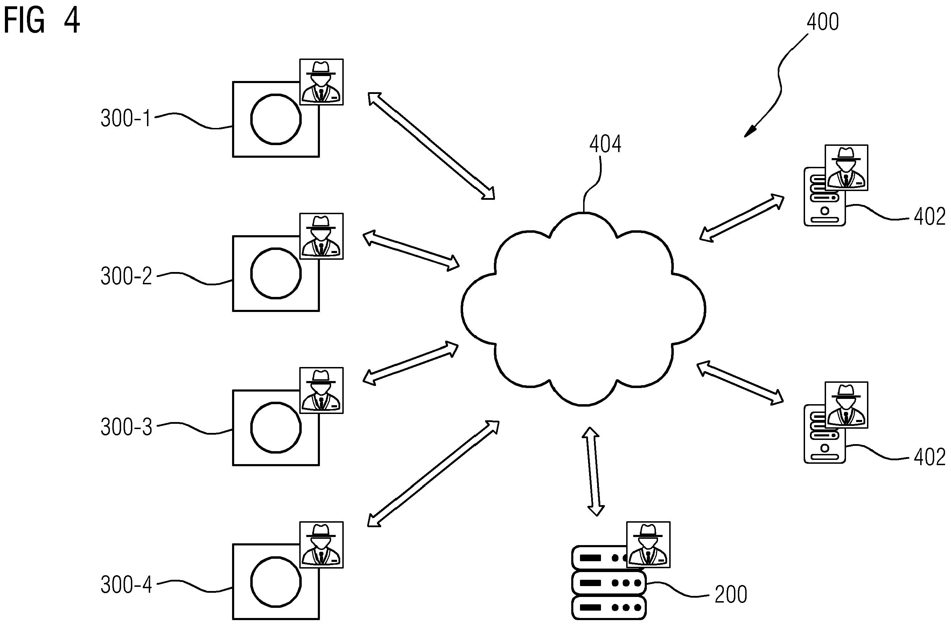

Fig. 4 zeigt ein Ausführungsbeispiel eines Systems 400. Das System 400 umfasst eine Scheduler-Agenten-Vorrichtung 200 und eine Mehrzahl Gerät-Agenten-Vorrichtungen 300.

In dem Ausführungsbeispiel der Fig. 4 kann beispielsweise die erste Gerät-Agenten-Vorrichtung 300-1 einem MRT zugeordnet sein, die zweite Gerät-Agenten-Vorrichtung 300-2 ebenfalls einem MRT zugeordnet sein, die dritte Gerät-Agenten-Vorrichtung 300-3 einem CT zugeordnet sein und die vierte Gerät-Agenten-Vorrichtung 300-4 einem weiteren bildgebenden Gerät zugeordnet sein, beispielsweise einem Röntgengerät.

Das System 400 in Fig. 4 umfasst ferner beispielhaft zwei Patienten-Agenten-Vorrichtungen 402 zur Zuordnung eines medizinischen Geräts aus einer Gruppe medizinischer Geräte zu einer Untersuchung eines Patienten.

Jede Patienten-Agenten-Vorrichtung 402 kann eine Empfangseinheit umfassen, die ausgebildet ist zum Empfangen mindestens eines Patientenparameters zur Untersuchung eines Patienten.

Ferner kann jede Patienten-Agenten-Vorrichtung 402 eine Sendeeinheit umfassen, die ausgebildet ist zum Senden, an die Scheduler-Agenten-Vorrichtung 200, einer Nutzungsanfrage zur Untersuchung des Patienten mittels eines medizinischen Geräts aus der Gruppe der medizinischen Geräte. Die Nutzungsanfrage kann den mindestens einen Patientenparameter des Patienten umfassen.

Die Anzahlen an Gerät-Agenten-Vorrichtungen 300 und Patienten-Agenten-Vorrichtungen 402 in Fig. 4 sind jeweils beispielhaft gezeigt. Andere Anzahlen der jeweiligen Vorrichtungsarten sind unabhängig voneinander möglich. Beispielsweise kann eine Radiologie-Abteilung eine Vielzahl an medizinischen Geräten und zugeordneten Gerät-Agenten-Vorrichtungen 300 umfassen. Alternativ oder ergänzend kann eine Radiologie-Abteilung beispielsweise eine (oder eine geringe Anzahl) zentralisierte Patienten-Agenten-Vorrichtung 402 umfassen. Weiterhin alternativ oder ergänzend kann die Scheduler-Agenten-Vorrichtung 200, beispielsweise über eine in Fig. 4 schematisch dargestellte Cloud-basierte Infrastruktur 404 mit einer Vielzahl Radiologie-Abteilungen, beispielsweise innerhalb einer Stadt, in Datenaustausch stehen.

Die Aufgabe, einen bestimmten Patienten zu einem bestimmten medizinischen Gerät (beispielsweise einem MRT-Scanner) zu leiten, wird im Ausführungsbeispiel der Fig. 4 an ein (z.B. cloudbasiertes) Zuordnungssystem (auch: Verhandlungssystem) 400 delegiert, das das am besten geeignete medizinische Gerät (insbesondere ein bildgebendes medizinisches Gerät, kurz: Scanner) für die individuelle diagnostische Bildgebungsanforderung bestimmt. Die Bestimmung des am besten geeigneten medizinischen Geräts beinhaltet die Analyse der erforderlichen Geräte- (auch: Scanner-) Kapazitäten auf der Grundlage vorbestimmter (und/oder wichtiger) Faktoren (auch: Kriterien).

Ein vorbestimmter (und/oder wichtiger) Faktor kann die Art einer Bildgebungsanforderung umfassen. Beispielsweise kann eine orthopädische Untersuchung eine bestimmte Ausstattung, z.B. MRT-Spulen (auch: Antennenspulen), und/oder spezielle (z.B. Scanner-) Software und/oder (insbesondere Software-) Lizenzen erfordern. Alternativ oder ergänzend kann eine hohe (z.B. Magnet-) Feldstärke für die beste Bildqualität bevorzugt werden.

Alternativ oder ergänzend kann ein vorbestimmter (und/oder wichtiger) Faktor eine Teilnahme des Patienten an einer Forschungsstudie umfassen. Beispielsweise kann ein Patient, der der Teilnahme an einer Forschungsstudie zugestimmt hat, an ein anderes medizinisches Gerät (auch: Scanner) weitergeleitet werden (z.B. als für eine herkömmliche Routineuntersuchung vorgesehen), wo zusätzliche Forschungspulssequenzen verfügbar sind.

Weiterhin alternativ oder ergänzend kann ein vorbestimmter (und/oder wichtiger) Faktor eine bestimmte Patientenbedingung umfassen. Beispielsweise wenn ein Patient Metallimplantate und/oder einen Herzschrittmacher hat, klaustrophobisch ist und/oder starkes Übergewicht hat, kann ein Teil der medizinischen Geräte (auch: Scanner) ungeeignet und/oder bestimmte medizinische Geräte (auch: Scanner) für die Untersuchung solcher Fälle vorzuziehen sein.

Weiterhin alternativ oder ergänzend kann ein vorbestimmter (und/oder wichtiger) Faktor Richtlinien für die Auswahl von medizinischen Geräten (auch: Scannern) durch die Einrichtung umfassen. Beispielsweise können Routineuntersuchungen, die mit medizinischen Geräten (auch: Scannern) mit einer herkömmlicherweise üblichen Ausstattung versehen sind, mit hohem Durchsatz durchgeführt werden.

Weiterhin alternativ oder ergänzend kann ein vorbestimmter (und/oder wichtiger) Faktor eine Verfügbarkeit und/oder einen Standort umfassen. Beispielsweise muss in Notfallpatient oftmals innerhalb von zwei Stunden untersucht (auch: gescannt) werden und/oder kann nicht in ein anderes Gebäude transportiert werden.

Jedes Kriterium (auch: Faktor) kann eine unterschiedliche Priorität haben. Alternativ oder ergänzend kann eine Kombination von bevorzugten Faktoren (und/oder Fähigkeiten des medizinischen Geräts) angegeben werden, beispielsweise durch die Patienten-Agenten-Vorrichtung 402. Die Aufgabe, einen bestimmten Patienten zu einem bestimmten medizinischen Gerät (z.B. einem MRT-Scanner) zu leiten, wird in dem in Fig. 4 gezeigten Ausführungsbeispiel an eine Cloud-basierte Verarbeitung delegiert, die das am besten geeignete medizinische Gerät (z.B. den Scanner) für die individuelle diagnostische Bildgebung bestimmt.

Erfindungsgemäß können zwei Hauptmodule implementiert werden. Ein erstes Hauptmodul umfasst einen Algorithmus, der die Eingangsvariablen (z.B. Bildgebungsanforderung des Patienten mit Randbedingungen und/oder institutionelle Richtlinien) in eine Liste von Anforderungen an die Fähigkeiten des medizinischen Geräts (auch: Scannerfähigkeiten) für den individuellen Fall übersetzt. Ein zweites Hauptmodul kann eine Cloud-basierte Aktivität ausführen, die eine Liste von medizinischen Geräten (auch: Scannern) identifiziert, die den Anforderungen für den individuellen Fall entsprechen. Die Identifizierung eines oder mehrerer medizinischer Geräte (auch: Scanner) kann durch den Einsatz des bedarfsgerechten Zuordnungssystems 400 (das auch als marktbasiertes Verhandlungssystem bezeichnet werden kann) mit Software-Agenten 200, 300 und 402 erreicht werden.

Die sich aus dem zweiten Hauptmodul ergebende Liste, insbesondere identifizierter medizinischer Geräte (auch: Scanner), kann einem Nutzer (beispielsweise Krankenhauspersonal) direkt als Eingabe für den Patientenplanungsprozess präsentiert werden. Alternativ oder ergänzend kann die sich aus dem zweiten Hauptmodul ergebende Liste in eine Patientenplanungssoftware integriert werden.

Insbesondere kann das zweite Hauptmodul erfindungsgemäß durch einen Agenten-basierten Zuordnungsmechanismus (auch: Verhandlungsmechanismus) realisiert werden.

Die Erstellung einer optimalen, oder nahezu optimalen, Zuordnung (z.B. umfassend einen Terminplan) für Patienten unter Berücksichtigung von Kriterien (auch: Faktoren), beispielweise der besten Eignung einer Art medizinischer Geräte (auch: Scannertyp), ihrer Verfügbarkeit und des geeigneten Standorts, ist herkömmlicherweise eine nicht-triviale und hochdynamische Aufgabe.

Um die Anforderungen an die Zuordnungen (z.B. umfassend Terminpläne) zu erfüllen, werden erfindungsgemäß Software-Agenten 200, 300 und 402 eingesetzt mit geeigneten Kommunikationsstrategien (die auch als Verhandlungsszenarien bezeichnet werden können).

Die Software-Agenten 200, 300 und 402 können unabhängige Software-Komponenten umfassen, die jeweils ihre eigenen Ziele verfolgen können. Sie können untereinander Nachrichten als Teil von Kommunikationsprotokollen (auch: Verhandlungsprotokollen oder Verhandlungsszenarien) austauschen, um ihre Ziele zu verfolgen.

Erfindungsgemäß wird eine offene bedarfsbasierte Kommunikationsplattform (die auch als marktbasierte Verhandlungsplattform bezeichnet werden kann) mit den folgenden Akteuren geschaffen, die jeweils durch einen (z.B. eine Software umfassenden) Agenten repräsentiert werden.

Eine Gerät-Agenten-Vorrichtung 300 (auch kurz: Gerät-Agent oder Scanner-Agent) ist einem medizinischen Gerät (auch: Scanner) zugeordnet bzw. repräsentiert dieses. Die, oder jede, Gerät-Agenten-Vorrichtung 300 verfügt über die entsprechenden Informationen, beispielsweise die Art des medizinischen Geräts (auch: Scannertyp), Standort, Diagnosefähigkeiten, und/oder Einschränkungen (z.B. hinsichtlich der bereitgestellten Software, Spezialzubehör und/oder zeitlicher Verfügbarkeit).

Eine Patienten-Agenten-Vorrichtung 402 (auch kurz: Patienten-Agent) vertritt die Interessen des Patienten, um die Anforderungen zu erfüllen, die auf einer Reihe von Patientenparametern, beispielsweise der Art der Untersuchung (auch: des Scans), Dringlichkeit und/oder Mobilität, basieren.

Eine Scheduler-Agenten-Vorrichtung 200 (auch kurz: Scheduler-Agent oder Planer-Agent) ist dafür verantwortlich, eine Zuordnung (z.B. umfassend einen Zeitplan) zu erstellen, die auf den Interessen des Nutzers (z. B. Krankenhäuser) basiert.

Die Interessen des Nutzers können den Kriterien (auch: Faktoren) entsprechen und/oder diese umfassen. Beispielsweise können die Interessen einen Zeitplan mit minimalen Verzögerungen für den Patienten, maximaler Auslastung der medizinischen Geräte (auch: Scannerauslastung), minimalen Bewegungsanforderungen für den Patienten, Einbeziehung von geplanten Ausfallzeiten von medizinischen Geräten (auch: Scannern) für Wartungsarbeiten und/oder die Fähigkeit, auf unvorhergesehene Ereignisse und/oder Störungen zu reagieren, umfassen. Die unvorhergesehenen Ereignisse und/oder Störungen können Störungen, beispielsweise im geplanten Arbeitsablauf, durch massive Notfalleinsätze (z. B. große Verkehrsunfälle, insbesondere auf der Autobahn) oder Störungen im Krankenhaus (z. B. Überschwemmung und/oder Feuer) umfassen.

Die Scheduler-Agenten-Vorrichtung 402, mehrere Gerät-Agenten-Vorrichtungen 300 und mehrere Patienten-Agenten-Vorrichtungen 402 sind beispielhaft in Fig. 4 gezeigt. In dem Ausführungsbeispiel der Fig. 4 kommuniziert die Scheduler-Agenten-Vorrichtung 402 mit den Gerät-Agenten-Vorrichtungen 300 und mit den Patienten-Agenten-Vorrichtungen 402 mittels einer Cloudbasierten Infrastruktur 404.

Jede Einheit - z.B. (insbesondere bildgebendes) medizinisches Gerät, Scheduler, Patient - kann durch eine (insbesondere eine Software umfassende) Agenten-Vorrichtung (kurz auch: Software-Agenten) 200; 300; 402 repräsentiert werden.

Die Agenten-Vorrichtungen 200; 300; 402 verfolgen jeweils ihre eigenen Ziele durch Verhandlung und/oder Nachrichtenaustausch mit anderen Agenten-Vorrichtungen 200; 300; 402. Die Rolle der Gerät-Agenten-Vorrichtungen 300; 300-1; 300-2; 300-3; 300-4 (kurz auch: Scanner-Agenten), z.B. wie in Fig. 4 gezeigt, kann darin bestehen und/oder umfassen, die relevanten Informationen über das medizinische Gerät (auch: Scanner), beispielsweise seine Fähigkeiten (z.B. umfassend eine Magnetfeldstärke eines MRT) und/oder seinen Standort zu verwalten.

Die Patienten-Agenten-Vorrichtungen 402 (kurz auch: Patientenagenten) können das Ziel verfolgen, den Patienten so schnell und bequem wie möglich unterzubringen.

Die Scheduler-Agenten-Vorrichtung 200 (kurz auch: Scheduler-Agent) kann versuchen, Zuordnungen (z.B. inklusive Zeitplänen) zu erstellen, um die Ziele der Patienten-Agenten-Vorrichtungen 402 zu erfüllen und gleichzeitig die Auslastung der medizinischen Geräte (auch: Scanner) zu maximieren (beispielsweise für die gesamte Gruppe und/oder Flotte von medizinischen Geräten einer Art und/oder eines Typs). Darüber hinaus kann sich die Scheduler-Agenten-Vorrichtung 200 um die dynamischen Elemente des Systems 400 kümmern und auf, insbesondere unvorhergesehene, Ereignisse (beispielsweise umgehend und/oder zeitnah) reagieren.

Die Kommunikationswege (auch: Kommunikationsszenarien oder Verhandlungsszenarien) zwischen den Agenten-Vorrichtungen 200; 300; 402 können im Voraus entworfen und/oder festgelegt sein. Die Kommunikationswege (auch: Verhandlungsszenarien) können die Art und/oder den Inhalt der ausgetauschten Nachrichten beinhalten.

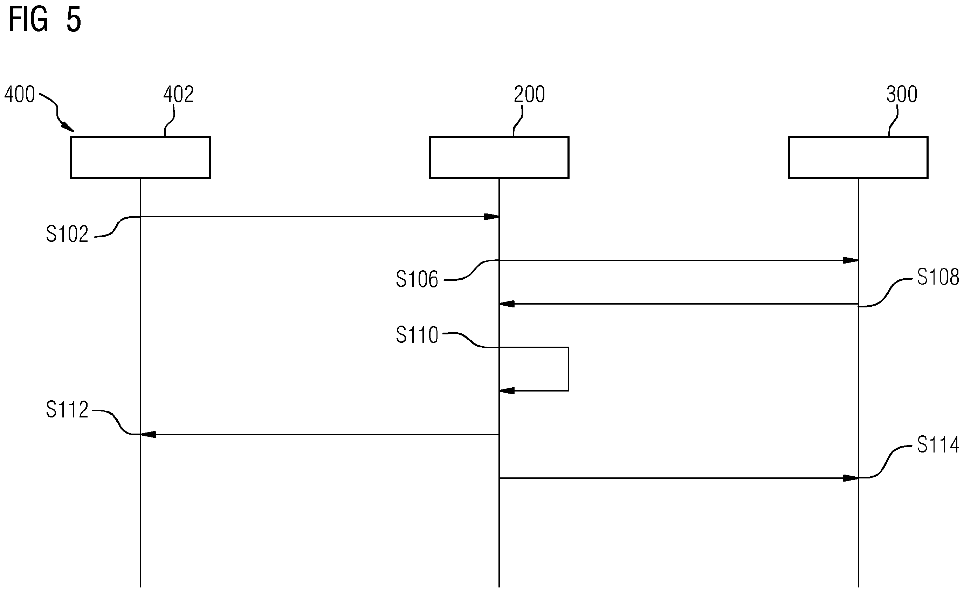

Ein Kommunikationsszenario kann die Anfrage des Patienten nach einer Zuordnung (z.B. umfassend einen Terminplan) auf der Grundlage bestimmter Kriterien (auch: Faktoren) darstellen, wie in Fig. 5 gezeigt.

In Fig. 5 sendet an Bezugszeichen S102 eine Patienten-Agenten-Vorrichtung 402 eine Nutzungsanfrage (die auch Zuordnungsanfrage genannt werden kann, und/oder z.B. umfassend eine Terminanfrage) auf der Grundlage angegebener Patientenparameter (auch: Patienteninformationen) an die Scheduler-Agenten-Vorrichtung 200. An Bezugszeichen S106 fragt die Scheduler-Agenten-Vorrichtung 200 die aktuelle Verfügbarkeit medizinischer Geräte von den jeweils zugeordneten Gerät-Agenten-Vorrichtungen 300 ab (beispielsweise von allen medizinischen Geräten und/oder Scannern) und erhält an Bezugszeichen S108 eine Verfügbarkeitsauskunft von jeder der angefragten Gerät-Agenten-Vorrichtungen 300 (beispielsweise von allen Gerät-Agenten-Vorrichtungen 300 und somit Informationen über die Verfügbarkeit aller medizinischer Geräte in der Gruppe und/oder Flotte).

An Bezugszeichen S110 erstellt die Scheduler-Agenten-Vorrichtung 200 dann eine neue Zuordnung (auch: Zuordnungsplan, beispielsweise umfassend einen Terminplan) auf der Grundlage beispielswiese der Verfügbarkeiten der medizinischen Geräte, des jeweiligen Gerätetyps (auch: Scannertyps), z.B. entsprechend der Anfrage der Patienten-Agenten-Vorrichtung 402, und/oder der Entfernung zum nächstgelegenen medizinischen Gerät (auch: Scanner). Nachdem die Zuordnung (z.B. umfassend den Zeitplan) erstellt wurde, wird er an die jeweilige (oder alle) Patienten-Agenten-Vorrichtung 402 an Bezugszeichen S112 und an die Gerät-Agenten-Vorrichtung 300 an Bezugszeichen S114 weitergeleitet.

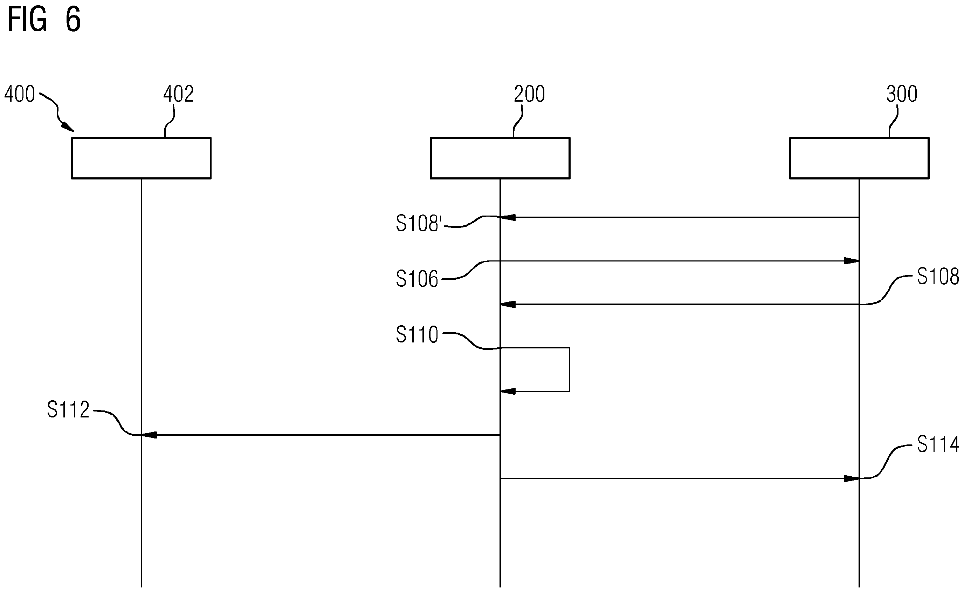

Eine weitere Art von Kommunikationsszenario umfasst unvorhergesehene Ereignisse, die beispielsweise ein (insbesondere bildgebendes) medizinisches Gerät (auch: Scanner) betreffen. Die Scheduler-Agenten-Vorrichtung 200 muss sofort reagieren, um die aktuelle Zuordnung (umfassend den aktuellen Zeitplan) umzuplanen, wie in Fig. 6 gezeigt.

Nach Erhalt der Information über ein geplantes Ereignis, z.B. eine regelmäßige Wartung, und/oder ein unvorhergesehenes Ereignis, z.B. einen Komponentenausfall und/oder eine Überschwemmung, von der Gerät-Agenten-Vorrichtung 300 an Bezugszeichen S108' fragt die Scheduler-Agenten-Vorrichtung 200 an Bezugszeichen S106 beispielsweise alle verfügbaren Zeitfenster für alle medizinischen Geräte (auch: Scanner) ab (z.B. für alle medizinischen Geräte eines Gerätetyps) und erhält an Bezugszeichen S108 die jeweilige Verfügbarkeitsauskunft.

Die Scheduler-Agenten-Vorrichtung 200 erstellt, beispielsweise analog zum Ausführungsbeispiel der Fig. 5, eine neue Zuordnung (auch: neuen Zeitplan) und teilt diese alle betroffenen Parteien mit, beispielsweise der oder den Patienten-Agentenvorrichtungen 402 an Bezugszeichen S112 und der oder den Gerät-Agenten-Vorrichtungen 300 an Bezugszeichen S114.

Die Erstellung von Zuordnungen (z.B. umfassend Zeitpläne) ist ein erfindungsgemäßer Aspekt. Die Scheduler-Agenten-Vorrichtung 200 erstellt eine Zuordnung (z.B. umfassend einen Zeitplan), die versucht, eine Reihe von Kriterien (auch: Faktoren) zu erfüllen, die von den Patienten-Agenten-Vorrichtungen 402 und Gerät-Agenten-Vorrichtungen 300 vorgegeben werden. Die Kriterien (auch: Faktoren) können beispielsweise die Art der für einen bestimmten Patienten erforderlichen Untersuchung (auch: Scan), die Entfernung zwischen Patienten und medizinischem Gerät, das gewünschte Zeitfenster für die Untersuchung (auch: Scan), den Grad der Auslastung eines bestimmten medizinischen Geräts (auch: Scanners), die Wartungsanforderungen und/oder die Behandlung unvorhergesehener Ereignisse wie Feuer, Überschwemmung und/oder Komponentenausfall umfassen.

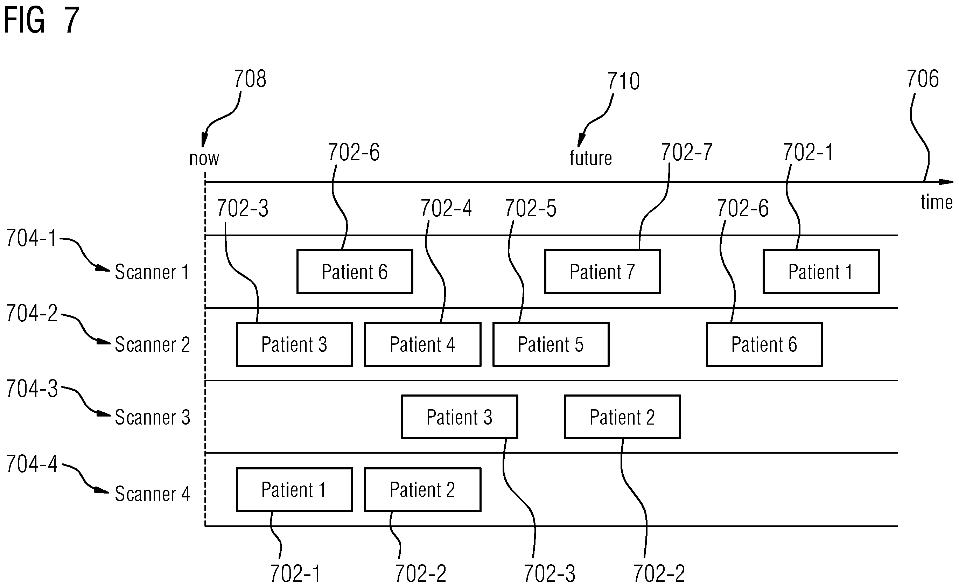

Fig. 7 zeigt beispielhaft eine Zuordnung (z.B. umfassend einen Zeitplan. In dem Ausführungsbeispiel in Fig. 7 sind vier medizinische Geräte ("Scanner") 704-1; 704-2; 704-3; 704-4 aufgelistet, denen entlang einer Zeitachse 706 Patienten 702-1; 702-2; 702-3; 702-4; 702-5; 702-6; 702-7 zugeordnet sind. Jedes medizinische Gerät 704-X (X=1,2,3,4) kann einer Gerät-Agenten-Vorrichtung 300-X zugeordnet sein. Alternativ oder ergänzend können die Patienten 702-X (X=1,2,3,4,5,6,7) durch eine oder mehrere Patienten-Agenten-Vorrichtungen 402 repräsentiert sein.

Das Ausführungsbeispiel der Fig. 7 für einen Zeitplan, der das Ergebnis der Kommunikation zwischen mehreren Gerät-Agenten-Vorrichtungen 300, mindestens einer Patienten-Agenten-Vorrichtung 402 und einer Scheduler-Agenten-Vorrichtung 200 ist, zeigt Zeitfenster von einem derzeitigen Zeitpunkt 708 bis in der Zukunft 710, wobei eine Reihe von erforderlichen (insbesondere Patienten- und/oder Geräte--) Parametern zugewiesen werden. Beispiele für die Parameter umfassen die Art des für einen bestimmten Patienten benötigten medizinischen Geräts (auch: Scanners), die Verfügbarkeit des medizinischen Geräts (auch: Scanners) und/oder seinen Standort.

Die Scheduler-Agenten-Vorrichtung 200 kann versuchen, einen Termin in der Zukunft zuzuweisen, der idealerweise den Anforderungen entspricht. Aufgrund begrenzter Ressourcen und manchmal unvorhergesehener Ereignisse wird dies allerdings nicht immer möglich sein.

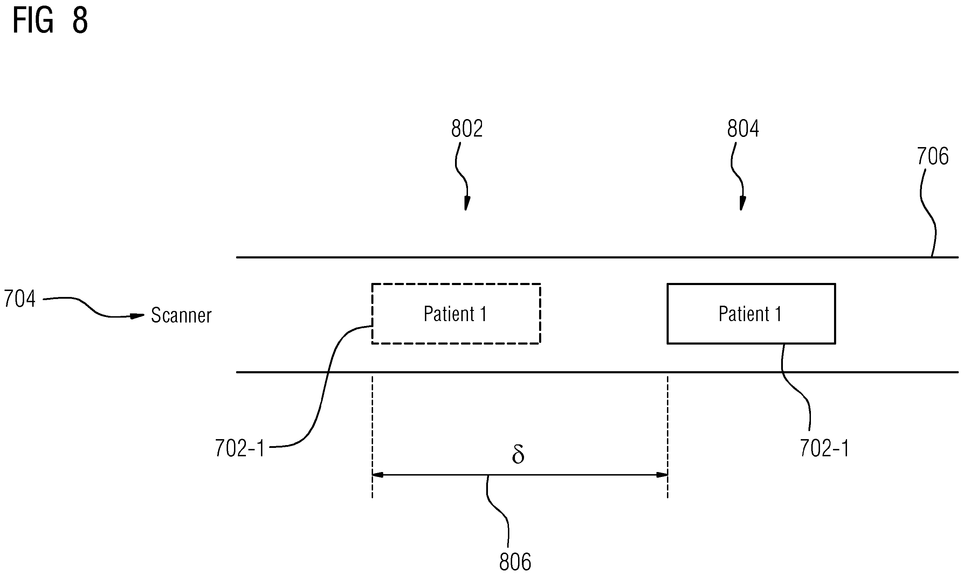

Ein wichtiger beispielhafter Faktor bei der Erstellung einer Zuordnung (z.B. umfassend einen Zeitplan) ist die in Fig. 8 gezeigte "Terminlücke" δ an Bezugszeichen 806 zwischen der idealen Position 802 des Zeitfensters und seiner (beispielsweise tatsächlich) geplanten Position 804.

Die beispielhafte "Terminlücke" δ an Bezugszeichen 806 umfasst einen einzelnen Wert (und/oder Parameter) einer linearen Kombination aller für den Zeitplan erforderlichen Parameter.

Die erforderlichen Parameter können auch als Kostenfunktionen bezeichnet werden. Jede Kostenfunktion kann bezüglich eines für den entsprechenden Parameter relevanten Wert normiert sein. Alternativ oder ergänzend kann jede Kostenfunktion dimensionslos sein.

Weiterhin alternativ oder ergänzend kann der einzelne Wert einer Gesamt-Kostenfunktion entsprechen. Zum Beispiel kann die Entfernung zwischen Patient und medizinischem Gerät (auch: Scanner) die "Terminlücke" 806 negativ beeinflussen. Ein früherer Termin in der Zukunft kann sich positiv auswirken, ebenso wie eine bessere Bildqualität des verfügbaren medizinischen Geräts (auch: Scanners).

Die Scheduler-Agenten-Vorrichtung 200 kann zunächst eine willkürliche Zuordnung (z.B. umfassend einen Zeitplan) erstellen. Beispielsweise werden nicht alle Termine zur idealen Zeit geplant.

Die Scheduler-Agenten-Vorrichtung 200 kann versuchen, einige der Termine zu tauschen, um die Terminlücke δ (und/oder die Kostenfunktionen zu mehreren Parametern, und/oder die Gesamt-Kostenfunktion) and Bezugszeichen 806 zwischen idealen und geplanten Terminen zu minimieren. Alternativ oder ergänzend kann die Scheduler-Agenten-Vorrichtung 200 versuchen, die Summe der Terminlücken (und/oder die Kostenfunktionen zu mehreren Parametern, und/oder die Gesamt-Kostenfunktion) 806 für alle Termine durch Tausch von Terminen zu minimieren.

Gemäß einem Ausführungsbeispiel können nur die Zuordnungen und/oder Termine getauscht werden, die die gleichen Anforderungen erfüllen, wie z.B. der gleiche Gerätetyp (auch: Scannertyp).

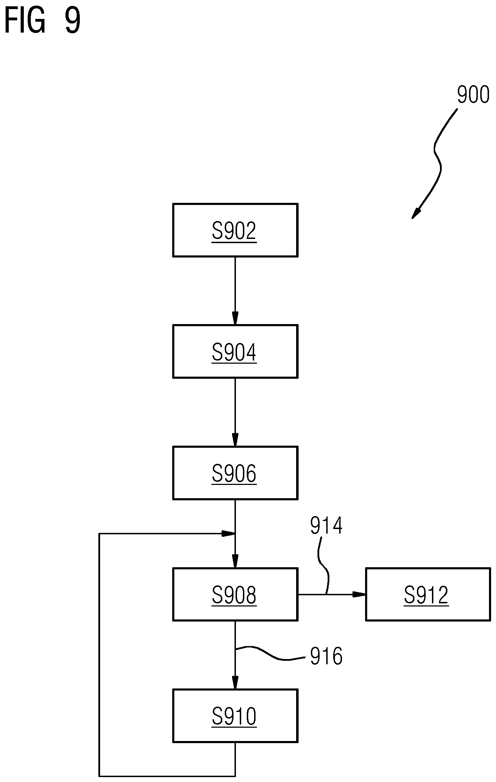

Fig.9 zeigt ein Ausführungsbeispiel eines Planungsalgorithmus 900 (auch: Optimierungsalgorithmus), der von der Scheduler-Agenten-Vorrichtung 200 ausgeführt werden kann.

Das beispielhafte Verfahren 900 der Fig. 9 beginnt an Bezugszeichen S902.

An Bezugszeichen S904 erstellt die Scheduler-Agenten-Vorrichtung 200 eine willkürliche Zuordnung (auch: Zeitplan), indem sie für alle Termine das nächste verfügbare Zeitfenster auswählt und dabei Anforderungen wie den passenden Gerätetyp (auch: Scannertyp) beachtet.

An Bezugszeichen S906 berechnet die Scheduler-Agenten-Vorrichtung 200 die anfängliche Summe aller Terminlücken und/oder Kostenfunktionen 806 für alle Termine.

An Bezugszeichen S908 versucht die Scheduler-Agenten-Vorrichtung 200 durch Vergleich aller Terminpaare und/oder Paare von Kostenfunktionen 806, ein solches Paar zu finden, bei dem die Summe aller Terminlücken und/oder Kostenfunktionen 806 minimiert werden kann (und/oder maximal reduziert werden kann).

Wenn an Bezugszeichen 916 ein solches Paar gefunden wird, wird an Bezugszeichen S910 fortgefahren. An Bezugszeichen S910 führt die Scheduler-Agenten-Vorrichtung 200 den Tausch innerhalb des Paares aus und berechnet die Summe aller Terminlücken und/oder Kostenfunktionen 806 neu. Anschließend wird wieder der Schritt S908 der Suche nach weiteren Paaren durchgeführt.

Wenn an Bezugszeichen 914 keine Verringerung der Summe aller Terminlücken und/oder Kostenfunktionen 806 gefunden wird, endet das Verfahren an Bezugszeichen S912. Die neue Zuordnung und/oder der neue Zeitplan werden den betroffenen Patienten-Agenten-Vorrichtungen 402 und Gerät-Agenten-Vorrichtungen 300 mitgeteilt, beispielsweise gemäß den Schritten S112 bzw. S114.

In jedem Ausführungsbeispiel kann die Kommunikation zwischen Bildanforderung (z.B. durch eine Patienten-Agenten-Vorrichtung 402) und einer Gruppe medizinischer Geräte (beispielsweise umfassend je eine Gerät-Agenten-Vorrichtung 300 pro medizinisches Gerät) über eine Scheduler-Agenten-Vorrichtung 200 als Kommunikationsschnittstelle (und/oder Verhandlungsschnittstelle) stattfinden. Insbesondere kann die Kommunikation Cloud-basiert sein.

Mittels der erfindungsgemäßen Technik kann eine Geräteauslastung (auch: Scannerauslastung) optimiert werden, insbesondere für Gruppen von (beispielsweise bildgebenden) medizinischen Geräten (auch: Scannerflotten) in größeren Einrichtungen und/oder mit einer Vielzahl an medizinischen Geräten. Die erfindungsgemäße Technik kann eine gute Skalierbarkeit ermöglichen, beispielsweise mit der Anzahl medizinischer Geräte (auch: Scanner) und/oder der Anzahl von Standorten (beispielsweise ohne größere Modifikationen bei Hinzufügen eines medizinischen Geräts und/oder eines Standorts).

In einem ersten Ausführungsbeispiel kann das medizinische Gerät einen MRT umfassen (und/oder die Modalität MRT umfassen).

Beispielsweise kann eine geplante und/oder angeforderte Untersuchung eine Knie-Untersuchung eines Patienten mit Beschwerden an einem künstlichen Kniegelenk umfassen.

Folgende Angaben können beispielsweise an den Patienten-Agenten übermittelt werden: eine Anforderung für eine Knieuntersuchung zur Abklärung von Beschwerden mit künstlichem Gelenkersatz, und ein Gewicht des Patienten, z.B. 210 kg, und/oder eine Angabe, dass der Patient stark fettleibig ist. Die Angaben können beispielsweise über eine spezielle Eingabemaske manuell eingegeben werden. Alternativ oder ergänzend kann es vorteilhafterweise eine Anbindung an ein Krankenhaus- oder/oder Radiologie-Informationssystem (KIS und/oder RIS) geben, in dem die Daten, insbesondere aus früheren Untersuchungen und/oder Behandlungen des Patienten, zumindest teilweise bereits vorliegen und/oder abgefragt werden.

Für eine Radiologie-Abteilung des Krankenhauses, an dem eine Untersuchung stattfinden soll, kann ein Algorithmus implementiert werden, mittels dem die Scheduler-Agenten-Vorrichtung 200 die Anforderung der Patienten-Agenten-Vorrichtung 402 in Anforderungen an ein geeignetes Gerät (auch: Scanner) übersetzt. Der Algorithmus kann Patientenparameter mit den Geräteparametern (auch: Scanner-Parametern), beispielsweise über relative Wichtungen (auch: Gewichte oder Gewichtungen), in Beziehung setzen. Z.B. kann eine Wichtung für die Beziehung von Patienten-Körpergewicht zur Geräte- (auch: Scanner-)) Feldstärke den Wert 0% haben, so dass das Körpergewicht für die Auswahl der Feldstärke nicht relevant ist. Alternativ oder ergänzend kann eine Wichtung von 100% bedeuten, dass die Parameterbeziehung zwingend eingehalten werden muss, z.B. kann ein Patientenparameter einer Anforderung einer Kontrastmitteluntersuchung erzwingen, dass das medizinische Gerät (auch: Scanner) über ein Kontrastmittelinjektionsgerät verfügt. Werte zwischen 0% und 100% können bedeuten, dass die Parameterbeziehung vorteilhaft aber nicht zwingend ist und so gut wie möglich übereinstimmen sollte, wenn abgewogen werden muss, welcher Patient besser an das eine oder andere medizinische Gerät (auch: Scanner) 'passt'.

Die Beziehungen zwischen Patientenparametern und Geräteparametern können in einer Übersetzungsmatrix zusammengefasst sein. Beispielsweise können die Einträge der Übersetzungsmatrix die Wichtungen umfassen.

Vorzugsweise ist die Übersetzungsmatrix von Patientenparametern zu Geräteparametern (auch: Scanner-Parametern) konfigurierbar, da sie abhängen kann von den Präferenzen der jeweiligen Institutionen (z.B. Krankenhaus und/oder Radiologie-Praxis) bzw. auch von Zeit zu Zeit Änderungen erforderlich sein können, beispielsweise bei einer Veränderung in der Gruppe der medizinischen Geräte und/oder in Richtlinien zu Untersuchungen mittels der medizinischen Geräte.

Für ein Patientenbeispiel, beispielsweise mit künstlichem Gelenkersatz und Übergewicht des Patienten, können sich beispielsweise folgende Anforderungen an das medizinische Gerät (auch: Scanner) ergeben: Messprotokolle für die Körperregion Knie müssen vorhanden (zwingende Anforderung für eine Knieuntersuchung); eine Ausstattung mit einer Lokalspule für das Knie muss vorhanden sein (zwingende Anforderung für eine Knie-untersuchung mittels MRT); die Anlage hat einen Tunneldurchmesser mindestens 70cm wegen Fettleibigkeit (zwingende Anforderung); die Patientenliege geeignet für 210kg (zwingende Anforderung); eine Feldstärke von 1,5 T (präferierte Anforderung, z.B. mit 80% Wichtung); die Anlage ist ausgestattet mit speziellen Untersuchungsprotokollen für Implantate (zwingende Anforderung bei künstlichem Gelenkersatz); das Bedienpersonal ist geschult bzgl. Untersuchungsmethoden und Vorsichtsmaßen bei Patienten mit Implantaten (zwingende Anforderung bei künstlichem Gelenkersatz).

Die Scheduler-Agenten-Vorrichtung 200 kann diese Anforderungen mit den Gerät-Agenten-Vorrichtungen 300 abgleichen und/oder verhandeln, um ein geeignetes medizinisches Gerät (auch: Scanner) zu identifizieren und bei einer Vielzahl von Patienten (beispielsweise mit Anforderungen, die von dem vorstehend detaillierten Patientenbeispiel abweichen) das Optimum zu erreichen.

In einem weiteren Ausführungsbeispiel kann ein medizinisches Gerät (auch: Scanner) einen Ausfall an die Scheduler-Agenten-Vorrichtung 200 melden. Die Meldung kann mittels der dem medizinischen Gerät zugeordneten Gerät-Agenten-Vorrichtung 300 erfolgen.

Beispielsweise kann das medizinische Gerät (auch: Scanner) defekt sein, so dass die Gerät-Agenten-Vorrichtung 300 an die Scheduler-Agenten-Vorrichtung 200 meldet, dass die für den nächsten Tag und/oder die nächsten 24h geplanten Patienten nicht untersucht werden können.

Die Scheduler-Agenten-Vorrichtung 200 kann aktiv werden und neue Zuordnungen und/oder Termine für die betroffenen Patienten suchen.

Alternativ oder ergänzend kann ein Patient dringend kurzfristig eine neue Zuordnung und/oder einen neuen Termin benötigen (beispielsweise aufgrund eines Unfalls) und hierfür die Zuordnung und/oder der Termin eines anderen, weniger dringenden Patienten (beispielsweise für ein Routine-Check-Up) zeitlich weiter nach hinten verlegt werden.

In der folgenden Tabelle sind beispielhafte Patientenparameter zusammengefasst, die in einer Patienten-Agenten-Vorrichtung 402 hinterlegt sein und/oder eingegeben werden können. In der letzten Spalte sind Anforderungen an Geräteparameter (auch: technische Anforderungen und/oder technische Parameter), insbesondere für MRT, beispielhaft aufgeführt in Abhängigkeit des jeweiligen Patientenparameters.

| **Patientenparameter, z.B. in einer Patienten-Agenten-Vorrichtung hinterlegt, und Eignungen medizinischer Geräte** | | |
|---|---|---|
| **Parameter** | **Typ. Werte (Auswahl/Beispiel)** | **Relevanz** |
| **Zu untersuchende Region** | Schädel / Herz / Abdomen / Hüfte / Knie / Fußgelenk, und/oder DRG-Code/ICD-10-Code/CPT-Code | Scanner muss Untersuchungen in dieser Körperregion unterstützen (z.B. durch entsprechende Messverfahren u. Auswerte-Software, geeignete MR-Spulen, ...) |
| **Untersuchungsparameter / Anforderung bzgl. diagnostischer Bildkontraste/ Abbildungsarten** | T1w / T2w / PD-FS / Angiographie / Kontrastmittelgabe und/oder CPT-Code | Scanner muss entsprechende Ausstattung aufweisen (dedizierte Mess-Sequenzen, Auswerte-Software, Kontrastmittel-Injektor, entsprechend geschultes Personal, ...) |
| **Patientengewicht** | 10-200 kg | Insbes. bei sehr schweren oder fettleibigen Patienten ergeben sich spezielle Anforderungen an den Scanner (Gewichtsbeschränkung der Patientenliegen, Durchmesser der Untersuchungsröhre) |
| **Patient ist klaustrophobisch** | Ja / Nein | |
| **Patient kann mehrere Minuten ruhig liegen** | Ja / Nein | |
| **Patient kann min. 15s die Luft anhalten** | Ja / Nein | Falls nein wird ein Scanner mit spezieller Ausstattung benötigt, die eine Messung bei freier Atmung erlaubt |
| **Erforderliche Feldstärke** | 0,5T, 1,5T / 3T / 7T / nicht relevant | Jede Feldstärke hat Vor- und Nachteile, die für unterschiedliche Messungen unterschiedlich zum Tragen kommen |
| **Patient hat metallisches Implantat** | Ja / Nein / Implantat-Typ | Für metallische Implantate eignen sich niedrigere Feldstärken besser als höhere. Weiterhin erzeugen metallische Implantate erhebliche Störungen des Magnetfeldes, so dass spezielle Messverfahren erforderlich sind, die in der Regel nicht auf jedem Scanner verfügbar sind. |
| **Untersuchung im Rahmen einer speziellen Studie** | Klinische Studie im Rahmen eines Forschungsprojektes / Entwicklung neuer Diagnoseverfahren | Scanner muss entsprechende Ausstattung aufweisen (dedizierte Mess-Sequenzen, Auswerte-Software, Kontrastmittel-Injektor, entsprechend geschultes Personal, ...), spezielles Zubehör, oft ist ein dedizierter Scanner erforderlich um Vergleichbarkeit der Ergebnisse zu gewährleisten. |
| **Zeitlicher Rahmen für die Untersuchung** | Innerhalb von 12h / 14 Tagen / im Zeitraum 13. August - 1. Sept. | Es muss ein geeigneter Scanner gefunden werden, der in diesem Zeitraum verfügbar ist |

In der folgenden Tabelle sind beispielhafte Geräteparameter (auch: Scannerparameter) zusammengefasst, die in einer Gerät-Agenten-Vorrichtung 300 hinterlegt sein können. Die Geräteparameter, insbesondere für MRT, können Eigenschaften und/oder Fähigkeiten des medizinischen Geräts umfassen, eine Ausstattung, beispielsweise mit Spezialzubehör, und/oder eine Verfügbarkeit.

| **Geräteparameter, z.B. in einer Gerät-Agenten-Vorrichtung hinterlegt, und Eignungen für Untersuchungen** | | |
|---|---|---|
| **Parameter** | **Typ. Werte** | **Relevanz** |
| **Feldstärke** | 0,5 / 1,5 / 3 / 7 Tesla | Höhere Feldstärken ermöglicht typischerweise höhere Bildqualität (höhere räumliche Auflösung, weniger Bildrauschen) |
| **Durchmesser der Patienten-öffnung** | 50 / 60 / 70 / 80 cm | Klaustrophobie, Untersuchung dickleibiger Patienten |
| **Max. Patienten-gewicht** | 200 kg / 300 kg | Gewichtsbegrenzung der Patientenliege |
| **Lokalspulen** | Dedizierte Spulen für Kopf / Hals / Wirbelsäule / Abdomen / Gelenke | Optimale Bildqualität erfordert praktisch immer eine Lokalspule (=Signalempfangseinheit), die der Körperregion angepasst ist. |
| **Sonderzubehör für spezielle Untersuchungen** | Injektor für Kontrastmittel, optischer Stimulator für fMRI, EKG-Sensor, verfahrbarer Patiententisch, Eignung für spezielle Eingriffe (Organpunktion, Tumorbiopsie, ...) | Spezialzubehör ist oft nur an einzelnen Geräten verfügbar (z.B. weil auf speziellen Scannertyp abgestimmt, hoher Preis, ...) |
| **Dedizierte Untersuchungsformen** | Schädel (Standard) Wirbelsäule (CWS,BWS,LWS) Wirbelsäule (Multi-Station) Knie (Standard, keine Implantate) Knie (Implantate) Angiographie (Kopf, Hals, peripher), und/oder unterstützte CPT-Codes und/oder DRG- bzw. ICD-10-Kategorien: z.B. "CPT 72159 - MR angio spine w/o&w/dye" | Messverfahren unterscheiden sich stark für unterschiedliche Körperregionen und diagnostische Fragestellungen -> der Scanner muss für die betreffende Untersuchung entsprechend geeignet bzw. ausgestattet sein (Hard- und Software). |
| **Dedizierte Software Lizenzen** | fMRI (funktionelle Gehirnuntersuchung) / Morphometrie-Paket / Implantatbildgebungsmethoden | Manche Untersuchungsmethoden sind an spezielle Software-pakete gebunden, die über Lizenz-Modelle nur auf entsprechenden Scannern verfügbar sind |
| **Verfügbarkeit** | Mo.-Fr. 7 - 20 Uhr | Anwesenheit von Fachpersonal, Nichtverfügbarkeit (z.B. Wartungszeiten, anderweitige Nutzung) |
| **Aktueller Status** | Frei / Verzögerungen bei aktueller Untersuchung / Belegung durch Notfallpatienten / nicht verfügbar wegen Defekt | In der Praxis treten häufig unvorhergesehene Verzögerungen, Vakanzen (Patient nicht erschienen) oder Blockaden (Notfallpatient, Anlagendefekt) auf |

Gemäß einem weiteren Ausführungsbeispiel kann das medizinische Gerät einen CT umfassen.

Bei CT als Modalität kann es beispielsweise folgende Patientenparameter und/oder Geräteparameter geben, die in einer Scheduler-Agenten-Vorrichtung 200 für die Zuordnung (auch: Zuteilung) der Patienten für bestimmte Untersuchungen berücksichtigt werden können.

Es gibt neben herkömmlichen Single-Source CT auch Dual-Source CTs, also Computertomographen mit einer bzw. zwei Röntgenquellen. Die Verwendung von zwei Röntgenquellen und Detektoren ermöglichen eine Verkürzung der Untersuchungszeiten, was insbesondere für Trauma-Patienten vorteilhaft sein kann. Alternativ oder ergänzend erlauben die Dual-Source CTs eine verbesserte zeitliche Auflösung und können damit bewegte Strukturen besser darstellen als herkömmliche Single-Source CTs. Eine verbesserte zeitliche Auflösung ist beispielsweise in der Kardio-Bildgebung sehr vorteilhaft und ermöglicht z.B. 4D Aufnahmen der Koronararterien.

Durch den technischen Fortschritt ist die erforderliche Röntgendosis immer geringer geworden. Abhängig von den Anforderungen an die Untersuchung ist nicht immer die niedrigste Dosis und damit nicht immer das neueste Gerät erforderlich. Bei Kindern und/oder Jugendlichen auf Grund der Langzeitfolgen der Röntgenstrahlen wird jedoch bevorzugt eine niedrige Röntgendosis verwendet. Alternativ oder ergänzend bevorzugt wird eine niedrige Röntgendosis (beispielsweise als Einzeldosis) für wiederholte Aufnahmen, beispielsweise wenn der Therapieerfolg mittels CT verfolgt wird.

Anforderungen an eine räumliche und/oder zeitliche Auflösung können auch Kriterien für die Auswahl eines CT Gerätes darstellen. Zum Beispiel kommt die hochauflösende Computertomographie bei Lungenerkrankungen bevorzugt zum Einsatz. Beispielsweise für Aufnahmen des schlagenden Herzens wird eine hohe räumliche und/oder zeitliche Auflösung benötigt.

Gemäß einem weiteren Ausführungsbeispiel kann das medizinische Gerät einen PET umfassen.

In der Onkologie bietet das PET (Positron-Emissions-Tomografie, beispielsweise als Modalität) beispielsweise wertvolle Informationen über Stoffwechselvorgänge in Tumorgewebe, die mit anderen Verfahren nicht möglich sind. PET-Geräte können beispielsweise die Stoffwechselrate von Glukose im Körper darstellen, wodurch sich insbesondere benigne und maligne Tumoren unterscheiden lassen können. Beispielsweise nehmen durch ihren erhöhten Glukose-Stoffwechsel maligne Tumoren (z.B. im Vergleich zu benignen Tumoren) mehr Kontrastmittel auf, das radioaktiv markierte Glukose mit kurzer Zerfallszeit enthält und dem Patienten vor der Untersuchung intravenös verabreicht wird. Alternativ oder ergänzend kann das PET-Verfahren die im Körper entstehende Strahlung räumlich aufgelöst messen.

Gemäß weiterer Ausführungsbeispiele sind Kombinationen von zwei oder mehr (insbesondere bildgebenden) medizinischen Geräten (auch: Scannern) möglich.

Eine Kombination von PET mit MRT oder CT in einem einzigen Gerät (z.B. als PET/MRT-Scanner bzw. PET/CT-Scanner bezeichnet) ermöglicht eine gemeinsame Nutzung der Vorteile der jeweiligen Modalität und/oder des jeweiligen Verfahrens (z.B. PET: Darstellung von Stoffwechselvorgängen, aber relativ niedrige räumlicher Auflösung; MRT: hoher Weichteilkontrast, aber relativ lange Messzeiten; und/oder CT: hohe Geschwindigkeit und hohe Auflösung, aber niedriger Weichteilkontrast).

Die Wahl (und/oder die Zuordnung) eines medizinischen Geräts durch die Scheduler-Agenten-Vorrichtung 200, beispielsweise aufgrund eines oder mehrerer Geräteparameter, kann von der Kombination der Gerätetypen (z.B. PET/MRT und/oder PET/CT) abhängen sowie von Patientenparametern, wie in folgender Tabelle beispielhaft gezeigt ist. Beispielsweise ist beim Vorhandensein eines Implantats eine Kombination PET/MRT als medizinisches Gerät üblicherweise nicht geeignet, während die Kombination PET/CT üblicherweise möglich ist.

| **Kombinationsmöglichkeiten verschiedener Gerätetypen und Anwendungsfälle** | | |
|---|---|---|
| **Parameter** | **Medizinischer Gerätetyp (auch: Scannertyp)** | **Kommentar** |
| **Patient mit Implantat** | PET/CT (-> PET/MRT nicht möglich!) | Implantat erzeugt starke Bildstörungen |
| **Beatmung oder Sedierung/Narkose erforderlich** | PET/CT | Erforderliche Geräte im Allgemeinen nicht MRT-Kompatibel |
| **Notfallpatient (zeitkritisch)** | PET/CT | CT deutlich schneller als MRT |
| **Röntgen Strahlung nicht vertretbar** | PET/MR | Zum Beispiel bei Kindern sollte jegliche Röntgenstrahlung vermieden werden und somit ist MRT zu bevorzugen |

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für die Zuordnung bildgebender medizinischer Geräte zu Untersuchungen von Patienten angewendet werden kann, sondern auch für andere medizinische Geräte, die zur zeitlich begrenzten Untersuchung und/oder Behandlung eines Patienten dienen. Des Weiteren können die Bauteile jeder Agenten-Vorrichtung (insbesondere der Scheduler-Agenten-Vorrichtung und der Geräte-Agenten-Vorrichtung), und/oder die Bauteile des Systems, auf mehreren physikalischen Produkten verteilt realisiert werden kann.

## Patentansprüche

1. Computer-implementiertes Verfahren (100) zur Zuordnung eines medizinischen Geräts aus einer Gruppe medizinischer Geräte zu einer Untersuchung eines Patienten, wobei das Verfahren in einer Scheduler-Agenten-Vorrichtung (200), implementiert ist, umfassend folgende Verfahrensschritte:
- Empfangen (S102), von einer Patienten-Agenten-Vorrichtung (402), einer Nutzungsanfrage zur Untersuchung eines Patienten mittels eines medizinischen Geräts aus der Gruppe der medizinischen Geräte, wobei die Nutzungsanfrage mindestens einen Patientenparameter des Patienten umfasst;
- Bestimmen (S104) einer Eignung mindestens eines medizinischen Geräts aus der Gruppe der medizinischen Geräte zur Untersuchung basierend auf dem mindestens einen empfangenen (S102) Patientenparameter und mindestens einem Geräteparameter des medizinischen Geräts;
- Zuordnen (S110) eines des mindestens einen als geeignet bestimmten (S104) medizinischen Geräts zu der empfangenen (S102) Nutzungsanfrage zur Untersuchung des Patienten basierend auf dem mindestens einen empfangenen (S102) Patientenparameter und dem mindestens einen Geräteparameter;
- Senden (S112), an die Patienten-Agenten-Vorrichtung (402), einer Mitteilung umfassend das zugeordnete (S110) als geeignet bestimmte (S104) medizinische Gerät zur Untersuchung des Patienten; und
- Senden (S114), an eine dem zugeordneten (S110) medizinischen Gerät beigeordnete Gerät-Agenten-Vorrichtung (300), einer Mitteilung umfassend die zugeordnete (S110) Untersuchung des Patienten.

2. Verfahren (100) nach Patentanspruch 1, wobei die Gruppe medizinischer Geräte eine Art medizinischer Geräte für eine vorbestimmte Art von Untersuchungen von Patienten umfasst.

3. Verfahren (100) nach einem der vorhergehenden Patentansprüche, wobei das medizinische Gerät ein bildgebendes Gerät umfasst, und/oder wobei die Gruppe medizinischer Geräte eine Gruppe bildgebender Geräte umfasst.

4. Verfahren (100) nach dem direkt vorhergehenden Patentanspruch, wobei das bildgebende Gerät, und/oder die Gruppe bildgebender Geräte, ausgewählt ist aus der Gruppe, bestehend aus:
- Einen Magnetresonanz-Tomograph, MRT;
- Einen Computer-Tomograph, CT;
- Ein Röntgengerät;
- Ein Ultraschallgerät;
- Einen Positronen-Emissions-Tomograph, PET, insbesondere ein Einzelphotonen-Emissions-Tomograph, SPECT; und
- Eine Kombination von zwei oder mehr bildgebenden Geräten, insbesondere eine Kombination von PET und CT, und/oder eine Kombination von PET und MRT.

5. Verfahren (100) nach einem der vorhergehenden Patentansprüche, ferner umfassend mindestens einen der folgenden Verfahrensschritte:
- Senden (S106) einer Verfügbarkeitsanfrage an mindestens eines des mindestens einen zur Eignung bestimmten (S104) medizinischen Geräts, insbesondere an die beigeordnete Gerät-Agenten-Vorrichtung (300); und
- Empfangen (S108; S108') einer Verfügbarkeitsauskunft des mindestens einen zur Eignung bestimmten (S104) medizinischen Geräts, insbesondere von der beigeordneten Gerät-Agenten-Vorrichtung (300).

6. Verfahren (100) nach einem der vorhergehenden Patentansprüche, wobei der mindestens eine Patientenparameter ausgewählt ist aus der Gruppe umfassend:
- - eine medizinische Fragestellung;
- eine zu untersuchende Körperstelle des Patienten;
- einen Körperbau des Patienten, insbesondere eine Größe und/oder ein Gewicht;
- ein Vorhandensein eines Implantats an oder nahe der zu untersuchenden Körperstelle des Patienten;
- ein Alter des Patienten;
- ein Geschlecht des Patienten;
- eine Mobilität des Patienten;
- eine psychische Fitness des Patienten:
- eine physische Fitness des Patienten;
- eine Dringlichkeit der Untersuchung des Patienten;
- eine Anforderung an einen Geräteparameter; und
- eine Teilnahme an einer klinischen Studie.

7. Verfahren (100) nach einem der vorhergehenden Patentansprüche, wobei der mindestens eine Geräteparameter ausgewählt ist aus der Gruppe, bestehend aus:
- einen Standort des medizinischen Geräts;
- eine Bauweise des medizinischen Geräts, insbesondere umfassend eine Röhre, halboffen und/oder gerüst-basiert;
- eine Belastbarkeit und/oder Verfahrbarkeit einer Patientenliege;
- das Vorhandensein von Spezialzubehör;
- eine Feldstärke, insbesondere eine Magnetfeldstärke;
- eine Anzahl an Röntgenquellen und zugehörigen Detektoren;
- eine Bildqualität;
- ein Vorhandensein von Spezial-Software zur Durchführung und/oder Auswertung der Untersuchung;
- eine durchschnittliche Zeitdauer einer Untersuchungsart, die der Nutzungsanfrage entspricht;
- eine zeitliche Verfügbarkeit;
- das Vorhandensein von geschultem Personal; und
- eine Eignung für klinische Studien.

8. Verfahren (100) nach einem der vorhergehenden Patentansprüche, wobei der Schritt des Empfangens (S102) eine Eingabe auf einer Benutzer-Schnittstelle und/oder eine Abfrage eines Datenbank-Systems umfasst.

9. Verfahren (100) nach einem der vorhergehenden Patentansprüche, wobei der Schritt des Zuordnens (S110) eines des mindestens einen als geeignet bestimmten (S104) medizinischen Geräts zu der empfangenen (S102) Nutzungsanfrage ein Optimieren einer Menge von Zuordnungen unterschiedlicher Patienten, umfassend den Patienten, zu medizinischen Geräten aus der Gruppe medizinischer Geräte umfasst.

10. Verfahren (100) nach dem direkt vorhergehenden Patentanspruch, wobei das Optimieren ein Minimieren einer Linearkombination dimensionsloser Kostenfunktionen umfasst, wobei jedem des mindestens einen Patientenparameters eine dimensionslose Kostenfunktion zugeordnet ist.

11. Verfahren (100) nach dem direkt vorhergehenden Patentanspruch, wobei die Linearkombination eine gewichtete Summe der dimensionslosen Kostenfunktionen umfasst.

12. Verfahren (100) nach einem der drei direkt vorhergehenden Patentansprüche, wobei das Optimieren einen oder mehrere Schritte eines paarweisen Vertauschens von Zuordnungen eines Patienten zu einem medizinischen Gerät umfasst.

13. Scheduler-Agenten-Vorrichtung (200) zur Zuordnung eines medizinischen Geräts aus einer Gruppe medizinischer Geräte zu einer Untersuchung eines Patienten, umfassend:
- eine Empfangseinheit (202-1), die ausgebildet ist zum Empfangen, von einer Patienten-Agenten-Vorrichtung, einer Nutzungsanfrage zur Untersuchung eines Patienten mittels eines medizinischen Geräts aus der Gruppe der medizinischen Geräte, wobei die Nutzungsanfrage mindestens einen Patientenparameter des Patienten umfasst;
- eine Bestimmungseinheit (204-1), die ausgebildet ist zum Bestimmen einer Eignung mindestens eines medizinischen Geräts aus der Gruppe der medizinischen Geräte zur Untersuchung basierend auf dem mindestens einen empfangenen Patientenparameter und mindestens einem Geräteparameter des medizinischen Geräts;
- eine Zuordnungseinheit (204-2), die ausgebildet ist zum Zuordnen eines des mindestens einen als geeignet bestimmten (S104) medizinischen Geräts zu der empfangenen Nutzungsanfrage zur Untersuchung des Patienten basierend auf dem mindestens einen empfangenen (S102) Patientenparameter und dem mindestens einen Geräteparameter;
- eine erste Sendeeinheit (202-4), die ausgebildet ist zum Senden, an die Patienten-Agenten-Vorrichtung, einer Mitteilung umfassend das zugeordnete als geeignet bestimmte medizinische Gerät zur Untersuchung des Patienten; und
- eine zweite Sendeeinheit (202-5), die ausgebildet ist zum Senden, an eine dem zugeordneten medizinischen Gerät beigeordnete Gerät-Agenten-Vorrichtung, einer Mitteilung umfassend die zugeordnete Untersuchung des Patienten.

14. Scheduler-Agenten-Vorrichtung (200) nach dem direkt vorhergehenden Patentanspruch, die ferner ausgebildet ist zur Durchführung des Verfahrens (100) nach einem vorhergehenden Verfahrensschritte.

15. Gerät-Agenten-Vorrichtung (300; 300-1; 300-2; 300-3; 300-4) zur Zuordnung eines medizinischen Geräts aus einer Gruppe medizinischer Geräte zu einer Untersuchung eines Patienten, wobei die Gerät-Agenten-Vorrichtung dem medizinischen Gerät zugeordnet ist, umfassend:
- eine erste Empfangseinheit (302-1), die ausgebildet ist zum Empfangen, von einer Scheduler-Agenten-Vorrichtung, einer Mitteilung umfassend die zugeordnete (S110) Untersuchung des Patienten.

16. Gerät-Agenten-Vorrichtung (300; 300-1; 300-2; 300-3; 300-4) gemäß dem direkt vorhergehenden Patentanspruch, ferner umfassend:
- eine zweite Empfangseinheit (302-3), die ausgebildet ist zum Empfangen einer Verfügbarkeitsanfrage betreffend das medizinische Gerät von der Scheduler-Agenten-Vorrichtung; und/oder
- eine Sendeeinheit (302-S), die ausgebildet ist zum Senden einer Verfügbarkeitsauskunft betreffend das medizinische Gerät an die Scheduler-Agenten-Vorrichtung.

17. System (400) zur Zuordnung eines medizinischen Geräts aus einer Gruppe medizinischer Geräte zu einer Untersuchung eines Patienten, umfassend:
- eine Scheduler-Agenten-Vorrichtung (200) gemäß einem der vorhergehenden Scheduler-Agenten-Vorrichtungsansprüche;
- eine Vielzahl von Gerät-Agenten-Vorrichtungen (300; 300-1; 300-2; 300-3; 300-4) gemäß einem der vorhergehenden Gerät-Agenten-Vorrichtungsansprüche, wobei jede Gerät-Agenten-Vorrichtung einem medizinischen Gerät zugeordnet ist; und
- mindestens eine Patienten-Agenten-Vorrichtung (402) zur Zuordnung eines medizinischen Geräts aus einer Gruppe medizinischer Geräte zu einer Untersuchung eines Patienten, umfassend:
- eine Empfangseinheit, die ausgebildet ist zum Empfangen mindestens eines Patientenparameters zur Untersuchung eines Patienten;
- eine Sendeeinheit, die ausgebildet ist zum Senden, an die Scheduler-Agenten-Vorrichtung (200), einer Nutzungsanfrage zur Untersuchung des Patienten mittels eines medizinischen Geräts aus der Gruppe der medizinischen Geräte, wobei die Nutzungsanfrage den mindestens einen Patientenparameter des Patienten umfasst.

18. Computerprogrammprodukt, umfassend Befehle, die bei der Ausführung des Programms durch einen Computer diesen veranlassen, das Verfahren (100) der vorhergehenden Verfahrensansprüche auszuführen.

19. Computerlesbares Speichermedium, umfassend Befehle, die bei der Ausführung durch mindestens einen Computer diesen veranlassen, die Schritte des Verfahrens (100) nach einem der vorhergehenden Verfahrensansprüche auszuführen.
